# FASCICULE DE BREVET EUROPEEN

(11) **EP 0 244 298 B1**
(45) Date de publication et mention de la délivrance du brevet: **30.11.1994**
(21) Numéro de dépôt: 87400884.0
(22) Date de dépôt: 16.04.1987
(51) Int. Cl.: C08B 37/10, A61K 31/725

(54) **Oligosaccharides hépariniques affinés pour les facteurs de croissance cellulaires**
Oligosaccharide von Heparin mit einer Affinität für Faktoren von Zellwachstum
Oligosaccharides of heparin having an affinity for cell growth factors

(30) Priorité: 17.04.1986 FR 8605546
(43) Date de publication de la demande: 04.11.1987
(73) Titulaire: SANOFI, 75008 Paris (FR)
(72) Inventeur: Lormeau, Jean-Claude, F-76150 Maromme (FR); Petitou, Maurice, F-75645 Paris Cedex13 (FR); Choay, Jean, F-75007 Paris (FR)
(74) Mandataire: Polus, Camille

(56) Documents cités:
- EP-A- 0 114 589
- EP-A- 0 140 781
- PROC. NATL. ACAD. Sci. USA, vol. 82, février 1985, pages 805-809; M. KLAGSBRUNet al.: "Heparin affinity of anionic and cationic capillary endothelial cellgrowth factors: Analysis of hypothalamus-derived growth factors and fibroblastgrowth factors"
- CARBOHYDRATE-RESEARCH, vol. 127, no. 1, avril 1984, pages 75-94, ElsevierScience Publishers B.V., Amsterdam, NL; A. LINKER et al.: "Structural studieson heparin. Tetrasaccharides obtained by heparinase degradation"

## Description

L'invention a pour objet des oligosaccharides de type héparinique (héparine ou héparane-sulfate) ainsi que leurs fragments, dotés d'une activité sur la division et la différentiation cellulaires, leur préparation et leurs applications thérapeutiques.

On sait que l'héparine, comme l'héparane-sulfate, sont des glycosaminoglycanes hautement hétérogènes. Ils sont ainsi constitués de chaînes formées d'une alternance de motifs sucre, à savoir de motifs de structure D-glucosamine et des motifs de structure acide uronique (acide L-iduronique ou acide D-glucuronique), ou l'inverse. Cette structure de base peut être régulière avec un enchaïnement de motifs disaccharidiques [D-glucosamine] - [acide L-iduronique], ou irrégulière, le motif acide uronique étant alternativement un motif acide L-iduronique et acide D-glucuronique.
De plus, le poids moléculaire de ces chaînes peut différer considérablement allant d'environ 2.000 à 50.000. En outre, la charge ionique varie, pour un motif de même structure, selon la teneur en groupes sulfates.

Ce caractère hétérogène se traduit par des différences de propriétés des chaînes selon les séquences qu'elles renferment.

Il est connu qu'environ un tiers des chaïnes d'héparine présente un site de liaison à l'ATIII (anti-thrombine III). De telles chaînes sont dotées d'une activité plus spécifique vis-à-vis de certains facteurs de la coagulation du sang.

Les 2/3 des chaînes d'héparine dépourvues du site de liaison à l'ATIII sont en revanche dépourvues de l'activité anticoagulante mettant en jeu ce site.

On a également rapporté l'affinité de certains facteurs de croissance cellulaires pour l'héparine. Cette affinité est utilisée pour purifier des facteurs de croissance cellulaires par chromatographie d'affinité sur des colonnes d'héparine-Sépharose®.

Les facteurs de croissance cellulaire qui présentent une affinité pour l'héparine sont des polypeptides anioniques ou cationiques, de poids moléculaire d'environ 12000 à 20000 possédant un rôle clé dans la physiologie cellulaire. Ils ont, en particulier, une activité sur la division et la différenciation cellulaires. Cette activité s'exerce spécialement sur certains types de cellules telles que les cellules fibroblastiques, les cellules endothéliales capillaires et certaines cellules musculaires. Ils sont impliqués également dans la différentiation des cellules neuronales.

Ces facteurs sont appelés FGF, abréviation pour "fibroblastic growth factor", ou encore ECGF, abréviation pour "endothelial cell growth factor" (voir les articles de J. Courty, Y. Courtois et D. Barritault dans Biochimie 1984, vol. 66, p. 419-428 et de Roy R. Lobb et James W. Fett dans Bio-chemistry. Vol. 23 n° 26, 1984, p. 6295-6299).

Comme illustrant la technique antérieure, on peut citer EP-A-0 140 781 qui décrit diverses héparines de faible poids moléculaire et en particulier des héparines à substitution NH₂ et EP-A-0 114 589 qui décrit des fragments d'héparine obtenus par dégradation par l'héparinase. Toutes ces héparines et fragments d'héparine possèdent des motifs acide D-glucuronique.

On notera que, dans la suite de la description et dans les revendications, le terme "oligosaccharide" ou encore celui de "glycosaminoglycane" recouvre les produits natifs, les fragments d'oligosaccharides obtenus par dépolymérisation de chaînes plus longues de glycosaminoglycanes, ainsi que les séquences oligo- ou polysaccharidiques telles qu'obtenues par voie de synthèse. Dans ces fragments et ces séquences, les motifs à l'extrémité réductrice et/ou à l'extrémité non réductrice peuvent être chimiquement modifiés par rapport au motif correspondant des chaïnes naturelles d'héparine ou d'héparane sulfate. Ces motifs modifiés seront toutefois également désignés par les termes motifs sucre ou unités oligosaccharidiques dans la suite de la description et dans les revendications.

De manière surprenante, les inventeurs ont constaté que les facteurs de croissance évoqués ci-dessus ne présentent une affinité élevée que pour certaines chaînes d'oligosaccharides. L'étude de cette affinité en vue de la séparation de telles chaînes a permis de mettre en évidence que des familles d'oligosaccharides homogènes au regard de leur poids moléculaire sont constituées en fait d'un mélange de nombreuses espèces anioniques de charges différentes et que les espèces les plus anioniques possèdent des activités régulatrices sur la division et la différenciation cellulaires, de haut intérêt sur le plan pharmacologique.

L'invention a donc pour but de fournir des oligosaccharides de type héparine ou héparane-sulfate présentant une affinité élevée pour les facteurs de croissance cellulaires capables de se fixer à l'héparine.

Elle vise également à fournir un procédé de préparation de ces oligosaccharides.

L'invention vise également les applications de ces oligosaccharides pour l'élaboration de médicaments actifs sur la division et la différenciation cellulaires.

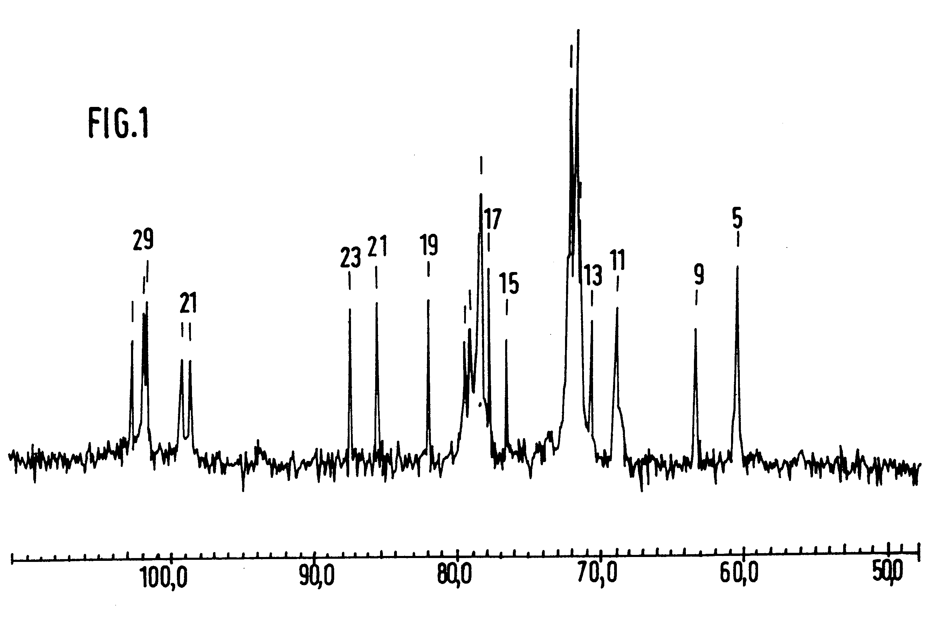

Les oligosaccharides de l'invention sont des produits essentiellement formés de chaînes
- ayant une affinité spécifique pour les facteurs de croissance cellulaires cationiques ou anioniques reconnaissant l'héparine,
- comprenant au moins une séquence de 5 unités monosaccharidiques conformes à celles présentes dans l'héparine naturelle et possédant une forte anionicité telle que celle caractérisée sur le spectre RMN représenté sur la figure 1, ainsi que les sels pharmacologiquement acceptables.

Les oligosaccharides de l'invention correspondent à la formule III suivante :

R(GH)ₙR' III

dans laquelle
- n est un nombre de 2 à 6,
- G-H correspond à un enchaînement disaccharidique de structure (acide L-iduronique 2-O-sulfate)-(D-glucosamine NH-sulfate 6-O-sulfate),
- G étant un radical d'acide L-iduronique 2-O-sulfate,
- H étant un radical de D-glucosamine NH-sulfate 6-O-sulfate,
- R représente soit un atome d'hydrogène, soit une unité dissacharidique G'-H dans lequel le radical G' est un radical d'acide uronique α,β insaturé,
- R' représente soit un atome d'hydrogène, soit une unité disaccharidique G-H' dans lequel le radical H' est un radical de 2,5-anhydromannitol ou d'acide 2,5-anhydromannonique,
étant entendu que les significations de R et R' sont telles que le nombre total d'unités monosaccharidiques des oligosaccharides, y compris les unités modifiées, est au plus égal à 14,
- capables de se fixer sur FGF anionique, fixé sur une colonne de chromatographie, en milieu tampon tris-HCl 0,01 M pH 7,4, 0,2 M NaCl,
et leurs sels pharmacologiquement acceptables.

D'une manière avantageuse, de tels oligosaccharides sont actifs sur le mécanisme de la division et la différenciation cellulaires sans qu'intervienne une activité sur le système de la coagulation, étant donné qu'ils sont dépourvus du site de liaison à l'ATIII.

L'enchaînement disaccharidique GH répond plus particulièrement à la structure II : 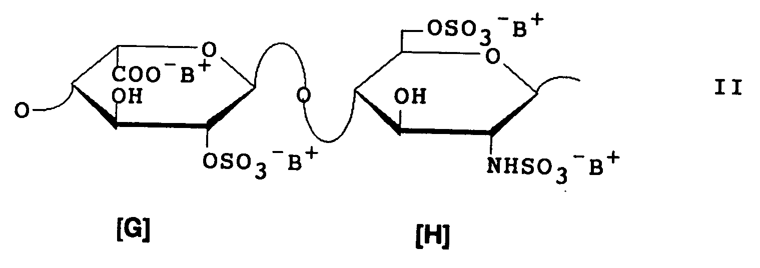
dans laquelle B⁺ représente un cation minéral ou organique donnant un sel physiologiquement acceptable.

Dans un groupe d'oligosaccharides de formule III ci-dessus, les extrémités non réductrices et les extrémités réductrices sont formées par une unité monosaccharidique (motif sucre) intacte, c'est-à-dire comportant un groupe -OH respectivement en position 4, ou sur le carbone anomère en position 1.

Dans un autre groupe, les oligosaccharides comportent à l'extrémité non réductrice et/ou à l'extrémité réductrice un groupe différent d'un motif sucre D-glucosamine ou acide L-iduronique.

Il s'agit plus spécialement d'une unité monosaccharidique (motif sucre) chimiquement modifiée par rapport à l'unité correspondante d'une chaîne héparinique.

Compte tenu des agents de dépolymérisaiton de l'héparine habituellement utilisés, à savoir l'acide nitreux ou une héparinase, l'unité à l'extrémité réductrice est une unité de structure 2,5-anhydromanno, à savoir un radical de 2,5-anhydromannitol ou d'acide 2,5-anhydromannonique et celui à l'extrémité non réductrice est un radical uronique α,β éthyléniquement insaturé.

Mais il est bien évident pour l'homme de l'art que, par voie de synthèse, il est possible d'introduire à chacune des extrémités divers radicaux organiques, dès lors qu'ils ne modifient pas les effets thérapeutiques des séquences saccharidiques définies ci-dessus.

Selon une disposition préférée de l'invention, les oligosaccharides, sont, en outre, homogènes au regard de leur degré de polymérisation c'est-à-dire du nombre d'unités constitutives de leurs chaïnes.

Un oligosaccharide particulièrement avantageux est constitué par un hexasaccharide.

Un tel hexasaccharide est formé de trois enchaïnements disaccharidiques répétés de structure [acide L-iduronique 2-O-sulfate]-[D-glucosamine-NH-sulfate, 6-O-sulfate]. Il s'agit du produit de formule III dans lequel R et R' représentent tous deux un atome d'hydrogène et n est égal à 3.

En variante, les unités à l'extrémité non réductrice et/ou à l'extrémité réductrice, sont chimiquement modifiées par rapport à l'unité correspondant présent dans l'héparine. Les hexasaccharides correspondants sont illustrés par la formule III dans laquelle R et/ou R' représentent une unité monosaccharidique modifiée.

Selon un autre aspect, l'hexasaccharide de l'invention correspond
- à un produit contenu dans une fraction correspondant à celle éluée entre un volume de 32 à 36 l dans un système de gel filtration où l'on dépose, en solution dans 500 ml de NaCl 0,5M, 60 g de précipité, obtenu par précipitation alcoolique d'un mélange de dépolymérisation nitreuse de l'héparine, sur une colonne de 100cm x 25cm contenant 45 litres d'agarose-acrylamide, équilibrée avec un tampon de NaCl 0,5 M, pH 6,0 et où l'on effectue l'élution à un débit de 1500 ml/heure,
- ce produit étant séparé par chromatographie d'affinité sur FGF anionique-Sépharose®, en utilisant une colonne de 2,5 cm x 6,5 cm, équilibrée en tampon tris-HCl 0,01 M, pH 7,4, 0,2 M NaCl renfermant 30 ml de FGF anionique-Sépharose ^{R}, sur laquelle on dépose 300 mg de la fraction ci-dessus en solution dans 60 ml du même tampon, en éluant à l'aide de ce tampon ajusté à 1 M de NaCl et en précipitant l'oligosaccharide de la fraction recueillie à l'aide d'éthanol.

Un autre oligosaccharide avantageux est constitué par un octosaccharide. Il s'agit plus spécialement d'un octosaccharide renfermant une séquence hexasaccharidique telle que définie ci-dessus. Par rapport à la structure correspondante trouvée dans l'héparine, l'octosaccharide est constitué d'unités non modifiées ou comporte, comme défini ci-dessus pour l'hexasaccharide, une unité modifiée, à l'extrémité non réductrice et/ou à l'extrémité réductrice.

Conformément à un autre aspect l'octosaccharide de l'invention est caractérisé en ce qu'il correspond :
- à un produit contenu dans une fraction correspondant à un volume d'élution entre 28 et 32 l dans un système de gel filtration où l'on dépose, en solution dans 500 ml de NaCl 0,5M, sur une colonne de 100 cm x 25 cm, contenant 45 litres d'agarose-acrylamide, équilibrée avec un tampon de NaCl 0,5 M, pH 6,0, 60 g de précipité, obtenu par précipitation alcoolique d'un mélange de dépolymérisation nitreuse de l'héparine, et où l'on effectue l'élution à un débit de 1500 ml/heure,
- ce produit étant séparé par chromatographie d'affinité sur FGF anionique-Sépharose®, en utilisant une colonne de 2,5 cm x 6,5cm, équilibrée en tampon tris-HCl 0,01 M, pH 7,4, 0,2 M NaCl renfermant 30 ml de FGF anionique-Sépharose®, sur laquelle on dépose 300 mg de la fraction ci-dessus en solution dans 60 ml du même tampon, en éluant à l'aide de ce tampon ajusté à 1 M de NaCl, et en précipitant l'oligosaccharide de la fraction recueillie à l'aide d'éthanol.

D'autres oligosaccharides avantageux sont constitués par des décasaccharides, dans lesquels les unités monosaccharidiques à l'extrémité réductrice ou à l'extrémité non réductrice sont chimiquement modifiées.

De tels décasaccharides sont caractérisés :
- en ce qu'ils sont contenus dans une fraction correspondant à un volume d'élution entre 25 et 28 l dans un système de gel filtration tel que rapporté ci-dessus pour l'obtention de l'octasaccharide, et qu'ils sont séparés par chromatographie d'affinité sur FGF anionique-Sépharose ® selon les conditions indiquées pour l'octasaccharide.

Les oligosaccharides formés de chaïnes renfermant 12 unités monosaccharidiques, le cas échéant chimiquement modifiées aux extrémités comme rapporté ci-dessus, sont également des produits avantageux.

Les oligosaccharides de l'invention peuvent se présenter sous forme de sels pharmacologiquement acceptables. On citera les sels de calcium, de sodium, de potassium et de magnésium.

L'invention vise également un procédé d'obtention des oligosaccharides définis ci-dessus.

Ce procédé est caractérisé par la mise en contact de préparations de glycosaminoglycanes de type héparine ou héparane-sulfate avec un facteur de croissance cellulaire anionique ou cationique avantageusement fixé à un support et par l'utilisation des tampons de forces ioniques définies afin d'éliminer les chaïnes dépourvues d'affinité et celles d'affinité moyenne pour le facteur de croissance, puis de récupérer les chaïnes saccharidiques fixées sur le facteur de croissance.

Ces dispositions permettent de séparer, d'un mélange, les chaînes de forte anionicité.

On utilise des préparations de glycosaminoglycanes de départ dont on élimine essentiellement les chaïnes ou les fragments ayant un site de liaison à l'AT III. Ces préparations sont formées de mélanges de chaïnes d'héparine ou d'héparane-sulfate natif ou de fragments de dépolymérisation de telles chaînes d'héparine ou d'héparane sulfate natif, ou de mélanges de chaïnes ou de fragments renfermant 14 motifs au plus.

La mise en contact des préparations de glycosaminoglycanes et du facteur de croissance cellulaire est réalisée avantageusement dans une colonne de chromatographie renfermant le facteur de croissance anionique ou cationique, équilibrée à l'aide d'un tampon. Le facteur de croissance cellulaire est avantageusement fixé à un support. Des supports appropriés sont à base de polysaccharides notamment d'agarose. Un support de ce type convenant dans cette application est constitué par du gel d'agarose commericalisé sous la marque Sepharose®.
On peut également utiliser des matrices hémocompatibles par exemple constituées de polystyrènes greffés, en particulier des supports décrits dans la demande de brevet FR 2534486 du 15/10/1982 au nom du Commissariat à l'Energie Atomique, et la demande de brevet FR 2553518 du 13/10/1983 aux noms de CHOAY S.A. et C.N.T.S.

Une séparation satisfaisante s'effectue en faisant passer une préparation de glycosaminoglycanes sur une matrice préalablement préparée sur laquelle est fixé du facteur de croissance cellulaire anionique ou cationique, équilibrée dans un tampon de force ionique correspondant à une concentration de 0,2 M de NaCl, puis en éluant la fraction oligosaccharidique retenue sur la colonne en ajustant le tampon à une force ionique correspondant à une concentration de 1 M à 2 M de NaCl.

Selon une disposition avantageuse de l'invention, l'opération de chromatographie d'affinité sur facteur de croissance cellulaire, est réalisée sur une fraction de glycosaminoglycanes homogène au regard de son poids moléculaire afin de séparer, d'une fraction donnée, un type de glycosaminoglycanes de caractéristiques déterminées.

Une fraction de glycosaminoglycanes de ce genre est obtenue avantageusement en soumettant une préparation de glycosaminoglycanes telle que définie ci-dessus à une opération de gel-filtration.

Par élution, à l'aide d'un tampon approprié, du mélange déposé au sommet d'une colonne de gel, on récupère successivement, en fonction de leurs poids moléculaires, des fractions d'oligosaccharides homogènes. On a recours aux gels utilisés de manière classique dans ce type d'opération, par exemple à des gels du type agarose-acrylamide.

Une séparation satisfaisante de ces fractions est obtenue en éluant avec un tampon de force ionique correspondant à celle d'un tampon de NaCl de 0,5M environ.

Les effluents de la colonne correspondant à la fraction de glycosaminoglycane souhaitée sont recueillis et la fraction est précipitée après addition d'un sel minéral par un solvant alcoolique, notamment l'éthanol. La fraction récupérée, homogène en ce qui concerne le poids moléculaire des chaînes, est en revanche très hétérogène au regard de la charge ionique.

La préparation de glycosaminoglycanes, formée du mélange de chaînes à fractionner puis à séparer par affinité pour les facteurs de croissance, ou à séparer directement à l'aide de ces facteurs sans effectuer de fractionnement, est plus spécialement obtenue par dépolymérisation de l'héparine, suivie d'un fractionnement afin de séparer les chaînes possédant le site de liaison à l'ATIII des chaînes dépourvues de ce site.

Les procédés de dépolymérisation connus sont basés notamment sur l'action de l'acide nitreux, de l'héparinase, de l'héparitinase ou du periodate sur l'héparine, la dépolymérisation nitreuse ou celle au periodate étant la plus intéressante pour une exploitation industrielle. L'héparine utilisée est une héparine brute ou de qualité pharmaceutique.

Les fragments de dépolymérisation mis en oeuvre peuvent posséder à l'extrémité non réductrice et à l'extrémité réductrice un motif modifié par rapport à la structure correspondante trouvée dans l'héparine : il s'agit respectivement d'un motif uronique α-β insaturé dans le cas d'une dépolymérisation à l'aide d'héparinase, d'héparitinase ou d'une α,β élimination, ou d'un motif de structure 2,5 anhydro-manno dans le cas d'une dépolymérisation à l'aide de l'acide nitreux.

Une technique de dépolymérisation nitreuse ménagée préférée correspond à celle faisant l'objet de la demande FR n° 2.503.714 du 10/04/1981 au nom de la demanderesse. Selon le procédé de cette demande, on fait réagir l'héparine et l'acide nitreux en milieu aqueux selon des concentrations respectives telles que tous les ions nitreux sont consommés lorsque le degré de dépolymérisation souhaité est atteint.

Avantageusement, les groupements réducteurs de structure 2,5-anhydromannose obtenus à l'issue de la déamination nitreuse sont ensuite réduits en groupes de structure 2,5-anhydromannitol par un agent réducteur tel que le tétrahydruroborate de sodium, ou bien sont oxydés en groupes de structure acide 2,5-anhydromannonique par un agent oxydant tel qu'un permanganate, notamment le permanganate de potassium.

Le fractionnement des chaînes affines et non affines, vis-à-vis de l'AT III, consiste avantageusement en un fractionnement alcoolique, plus spécialement un fractionnement éthanolique, en présence d'un sel minéral, conduisant à :
- un sous-ensemble constitué des fragments de taille correspondant principalement à 10, 12, 14, 16, 18 unités saccharidiques de l'héparine, possédant le motif pentasaccharidique de liaison à l'antithrombine III, et doué d'une importante activité anti-facteur Xa, et d'une faible activité anticoagulante globale,
- un sous-ensemble constitué de fragments de taille correspondant principalement à 2, 4, 6, 8, 10 unités saccharidiques de l'héparine, pratiquement dépourvus d'activité anticoagulante globale et présentant une très faible activité anti-facteur Xa.

Selon une variante, les glycosaminoglycanes de l'invention sont obtenus par échange d'ions avec des échangeurs d'anions basiques forts tels que des ammoniums quaternaires, en faisant varier selon un gradient approprié la force ionique du tampon d'élution.

L'utilisation d'un tampon d'équilibrage et de rincage puis d'un tampon d'élution ayant respectivement des forces ioniques correspondant à celles d'un tampon NaCl 0,5 M ± 0,1 M, puis à un tampon NaCl 1 à 2 M permet de séparer de manière satisfaisante les chaïnes affines recherchées du mélange.

Une séparation satisfaisante de chaïnes saccharidiques de type glycosaminoglycane, de forte anionicité, telles que définies ci-dessus, est obtenue en faisant varier la force ionique du tampon d'équilibre d'une valeur correspondant à une concentration de 0,4M de NaCl à une valeur correspondant à une concentration de 1M, le gradient étant adapté au type de glycosaminoglycanes à séparer, notamment à la longueur de leurs chaïnes.

L'étude pharmacologique des glycosaminoglycanes de l'invention a mis en évidence leurs propriétés sur la division et la différenciation cellulaires.

On sait que les facteurs de croissance cellulaire à affinité pour l'héparine agissent en particulier en se fixant sur des récepteurs cellulaires de plusieurs catégories que l'on trouve dans différents types cellulaires.

Comme l'ont constaté les inventeurs, les glycosaminoglycanes de l'invention modifient l'activité de ces facteurs de croissance.

Certaines cellules peuvent être autocrines (au sens défini dans Sporn M. B., Todaro G. J., New Engl. J. Med., 303, 878-880, 1980) et permettent de mettre en évidence le potentiel modulateur, à savoir activateur ou inhibiteur (notamment selon le phénotype, l'état de différenciation des cellules considérées), des glycosaminoglycanes de l'invention sur la division et la différenciation cellulaires, sans adjonction de facteur de croissance.

Des essais ont été réalisés dans des modèles expérimentaux particuliers utilisant des cultures de cellules endothéliales de capillaires de souris, de boeuf ou de veine ombilicale humaine. Ils ont montré une modulation de la multiplication cellulaire par rapport à une culture témoin dépourvue de glycosaminoglycanes, ou par rapport à un hexasaccharide de référence dépourvu d'affinité pour le FGF anionique.

On connaït également le rôle inhibiteur de la néoangiogénèse de certains stéroïdes en présence de fragments d'héparine. Des glycosaminoglycanes de l'invention, administrés avec des corticoïdes, ont avantageusement montré une action de ce type dans le modèle expérimental de la membrane chorioallantoïque de poulet décrit par J. Folkman et al dans Science, 221, 719 (1983)

Ces propriétés avantageuses s'accompagnent d'une grande innocuité.

L'invention vise donc l'utilisation des glycosaminoglycanes de l'invention en tant que modulateurs de la prolifération des cellules endothéliales; ils peuvent agir soit, à plus faibles doses, comme inhibiteurs dans les processus où la néoangiogénèse est pathologique, soit, à plus fortes doses, comme stimulateurs de la croissance dans certains processus où la protection des facteurs de croissance est nécessaire et où une régénération est souhaitée.

A titre d'exemple de processus de néoangiogénèse pathologique, on peut citer les rétinopathies des diabétiques, le développement des métastases, le psoriasis.

Les produits de l'invention peuvent également être utilisés pour inhiber le développement embryonnaire.

On peut aussi envisager l'utilisation des produits de l'invention pour stimuler la régénération dans certains cas de lésions endothéliales ou dans des cas de nécroses consécutives à des troubles circulatoires, ulcères variqueux par exemple. On peut également envisager leur emploi pour des lésions d'origine traumatique ou congénitale ou dans des pathologies de dégénérescence acquise, particulièrement lésions du tissu cardiaque après accidents d'origine vasculaire, telles que des infarctus.

L'invention vise également les compositions pharmaceutiques renfermant dans leur principe actif une quantité efficace d'au moins un glycosaminoglycane tel que défini ci-dessus, en association avec un véhicule pharmaceutique.

Des compositions pharmaceutiques avantageuses renferment l'hexasaccharide hautement anionique décrit plus haut, ou encore l'octosaccharide, le décasaccharide, ou le dodécasaccharide, ou un mélange les renfermant, sous forme de leurs sels pharmacologiquement acceptables.

Le principe actif de ces médicaments comprend le glycosaminoglycane seul ou, en variante, associé à un facteur de croissance cellulaire ayant de l'affinité pour l'héparine. Selon une autre variante, le glycosaminoglycane et le facteur de croissance sont utilisés en même temps sans association préalable.

Dans un autre mode d'utilisation avantageux des glycosaminoglycanes de l'invention, on peut utiliser ces derniers en association avec un stéroïde.

Compte tenu de leurs propriétés sur la division et la différenciation cellulaires, ces compositions pharmaceutiques sont utilisables principalement dans les indications thérapeutiques suivantes :
- stimulation de la réparation des tissus musculaires après des lésions, en particulier d'origine traumatique ou congénitale; du tissu cardiaque après des lésions d'origine vasculaire, telles que des infarctus; des tissus cutanés après des lésions notamment d'origine traumatique,
- accélération de la cicatrisation après des lésions d'origine traumatique,
- seuls ou en association avec des stéroïdes, inhibition des processus où la néoangiogénèse est pathologique, tels que rétinopathies des diabétiques, développement des métastases, psoriasis.

Les compositions pharmaceutiques de l'invention sont administrables sous différentes formes.

Ces formes pharmaceutiques peuvent être hydratées lorsque le glycosaminoglycane est utilisé seul pour la constitution du principe actif. Elles doivent être anhydres lorsque le principe actif comprend un facteur de croissance. Ce dernier est présent à raison de 1 à 200 µg par unité de prise, avantageusement de 25 à 100 µg.

Pour l'administration par voie orale, on a recours en particulier à des gélules, tablettes, comprimés, pilules, liposomes. Ces préparations renferment avantageusement de 50 mg à 5 g par unité de prise, de préférence de 50 à 250 mg pour les gélules, tablettes et pilules.

D'autres formes d'administration de l'invention sont constituées par des sprays, des pommades, des crèmes ou éventuellement des aérosols, la concentration en produit actif étant de l'ordre de 0,5 à 10%.

De façon avantageuse, les produits de l'invention sont administrés sous forme de compositions pharmaceutiques injectables par voie intraveineuse, sous-cutanée ou intra-musculaire, dans des solutions stériles ou stérilisables.

De façon avantageuse, les produits de l'invention sont utilisés en association avec des facteurs de croissance, en particulier dans des préparations à usage topique.

Les solutions destinées aux voies intraveineuse, sous-cutanée ou intra-musculaire renferment avantageusement de 10 à 250 mg/ml de compositions de glycosaminoglycanes, de préférence de 10 à 100 mg/ml, par exemple de 100 mg/ml, lorsque ces solutions sont destinées à l'injection par voie sous-cutanée. Elles peuvent contenir par exemple de 10 mg à 500 mg, notamment 150 mg/ml de glycosaminoglycanes, lorsqu'elles sont destinées à l'injection par voie intraveineuse ou par perfusion.

Avantageusement, de telles préparations pharmaceutiques sont présentées sous la forme de seringues non récupérables, prêtes à l'emploi.

Afin d'illustrer l'invention, on indique, ci-après, un exemple de posologie utilisable chez l'homme : cette posologie comprend, par exemple, l'administration au patient d'environ 30 à 500 mg/ jour en une ou plusieurs prises, par voie sous-cutanée ou intraveineuse. Par voie intraveineuse, on administre environ 150 mg/j, sachant que, par perfusion, les quantités injectées peuvent être de plusieurs dizaines de ml. Ces administrations sont effectuées de manière discontinue à intervalles réguliers, ou continues par perfusion. Ces doses peuvent être naturellement ajustées pour chaque patient en fonction des résultats et des analyses effectuées auparavant, la nature des affections dont il souffre, et, d'une manière générale, son état de santé.

Les compositions pharmaceutiques décrites ci-dessus peuvent comprendre, en outre, un composé de type stéroïde selon des quantités permettant l'administration de doses tolérables au patient.

En particulier, la quantité de stéroïde peut être telle que la dose administrée au patient n'excède pas 5 mg/kg/jour.

Dans d'autres compositions, cette dose est supérieure. Ces doses peuvent toutefois être élevées lorsqu'il s'agit de dérivés de stéroïdes ayant perdu leur activité hormonale.

L'invention concerne encore les réactifs biologiques dont les principes actifs sont constitués par les compositions de glycosaminoglycanes telles que définies plus haut. Ces réactifs biologiques peuvent être utilisés comme références ou étalons dans des études des éventuelles propriétés antitumorales d'autres substances, notamment, dans des tests mettant en jeu les techniques qui ont été décrites dans les exemples précédents.

Les glycosaminoglycanes de l'invention peuvent être fixés d'une manière conventionnelle à des gels insolubles et à d'autres matrices ou supports insolubles conventionnels inertes, pour fournir des supports de chromatographie d'affinité permettant d'effectuer des séparations. Comme supports appropriés, on citera ceux constitués par un polysaccharide notamment un gel d'agarose tel que celui commercialisé sous la marque Sépharose® et d'une manière générale des supports insolubles hydrophiles, possédant des greffons aminoéthyle, telles que l'aminoéthyl-cellulose, l'aminoéthyl-polyacrylamide, l'aminoéthyl-agarose. Les glycosaminoglycanes sont fixés sur ces supports par condensation, par exemple en milieu aqueux basique en présence de cyanoborohydrure de sodium, selon le schéma de réaction suivant : 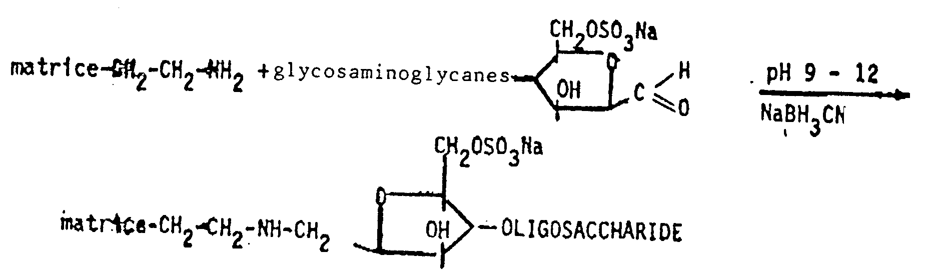
Les matrices ainsi obtenues sont conditionnées en colonne.

En mettant en contact, les glycosaminoglycanes de l'invention fixés sur des supports ou matrices, comme défini ci-dessus, avec des facteurs de croissance cellulaires anioniques ou cationiques, on dispose de moyens permettant de retenir sélectivement des facteurs de croissance cellulaires. Ces glycosaminoglycanes fixés sont ainsi utilisables comme ligands pour purifier des facteurs de croissance cellulaire ou pour réduire la concentration de ces facteurs dans un milieu donné.

D'une manière avantageuse, la séparation des facteurs de croissance est réalisée comme suit :
- L'extrait brut ou la solution contenant le facteur de croissance cellulaire à retenir est percolé sur la colonne, à pH neutre en tampon de faible force ionique : 0,1 à 0,6 M NaCl. La colonne est rincée par ce même tampon jusqu'à absence de protéines dans l'effluent. Le facteur de croissance cellulaire retenu sur la matrice est élué soit directement par un tampon de pH neutre et de force ionique 0,8 à 2 M NaCl, soit par un gradient continu de force ionique allant de 0,8 M à 2 M NaCl.

Ces facteurs de croissance peuvent provenir soit directement de milieux biologiques, soit de préparations ayant subi des opérations de purification préalables.

D'autres caractéristiques et avantages de l'invention sont rapportés dans les exemples qui suivent relatifs à la préparation de différents glycosaminoglycanes et aux résultats d'essais pharmacologiques.

On se réferera également aux figures 1 à 13, qui représentent respectivement,
- la figure 1, le spectre de RMN du ¹³C d'un hexasaccharide fortement anionique;
- la figure 2, le profil d'élution correspondant à la gel-filtration d'un mélange de dépolymérisation nitreuse;
- la figure 3, le profil d'élution de la fraction hexasaccharidique recueillie, lors de l'étape de gel filtration, en échange d'ions;
- la figure 4, le profil d'élution en échange d'ions de l'hexasaccharide isolé par chromatographie sur FGF-Sépharose®;
- la figure 5, le profil d'élution en chromatographie d'échange d'ions de l'hexasaccharide obtenu par chromatographie sur une colonne échangeuse d'anions,
- la figure 6, le profil d'élution en échange d'ions d'un mélange d'octasaccharides;
- la figure 7, le spectre de RMN du ¹³C de ce mélange d'octasaccharides;
- la figure 8, le profil d'élution en échange d'ions d'un mélange de décasaccharides;
- la figure 9, le spectre de RMN du ¹³C de ce mélange de décasaccharides;
- les figures 10 à 13, les courbes de la radioactivité en cpm en fonction de la dose de produit testé en ng obte nues respectivement avec le FGF anionique seul, un hexasaccharide seul ou avec FGF anionique, un autre hexasaccharide seul ou avec FGF anionique, un produit de contrôle dépourvu d'affinité pour le FGF anionique, utilisé seul ou avec du FGF anionique, et
- la figure 14, A-D représentent les courbes de prolifération de cellules endothéliales humaines en culture dans différentes conditions.

### EXEMPLE 1 :

### A - DEPOLYMERISATION

500 g d'héparine injectable, sous forme de sel de sodium, sont mis en solution dans 4500 ml d'eau déminéralisée, à un température de 18° C (concentration environ 11% P/V).

La solution obtenue est mise sous agitation énergique, et son pH est abaissé à 2,5 par addition d'acide chlorhydrique concentré. On ajoute alors 15 g de nitrite de sodium dissous dans 300 ml d'eau. (q.s.p.0,043 M final). Le pH de la réaction est ajusté à 2,5 par de l'acide chlorhydrique concentré, et le volume total de la solution est amené à 5000 ml afin d'avoir une concentration finale en héparine de 10% P/V. On laisse la réaction s'effectuer pendant 45 minutes. Ce laps de temps écoulé, l'absence d'ions nitreux résiduels dans la solution réactionnelle est vérifié au moyen de papier indicateur imprégné d'iodure de potassium amidonné (développement d'une coloration bleu-violacée en présence d'ions NO₂⁻).

Si des ions nitreux sont détectés, on laisse la réaction se poursuivre encore, jusqu'à disparition totale de ces ions et absence de réaction au papier iodo-amidonné, en effectuant des contrôles toutes les 3 ou 4 minutes.

Lorsque ces contrôles deviennent négatifs, la réaction est considérée comme étant achevée. Le pH de la solution est alors élevé à 10 à l'aide de soude concentrée, et l'on ajoute 5 g de tétrahydruroborate de sodium.

La solution est maintenue sous agitation pendant 15 heures.

Le tétrahydruroborate de sodium n'ayant pas réagi, est détruit par abaissement du pH à 3,0 à l'aide d'acide chlorhydrique concentré; la solution est soumise à agitation 15 minutes, puis on réajuste le pH à 7,0 au moyen de soude concentrée.

Pour récupérer les produits de la réaction, on ajoute 10 l d'éthanol, on laisse snuite au repos 48 heures, on laisse décanter le mélange puis on élimine le surnageant.

### B - FRACTIONNEMENT

Le précipité est redissous dans 9 litres d'eau déminéralisée (concentration 5% P/V en se basant sur le poids d'héparine de départ). On ajoute 100 g de chlorure de sodium, et l'on abaisse le pH de la solution à 3,8 à l'aide d'acide chlorhydrique concentré. Le volume est ajusté exactement à 10 litres à l'aide d'eau déminéralisée, et on ajoute sous agitation énergique 10 litres d'éthanol. On laisse reposer 48 heures. On siphonne le surnageant et on ajuste le pH à 7,0 à l'aide de soude 5N. On ajoute 19 litres d'éthanol. On laisse reposer 48 heures. On siphonne le surnageant pour le séparer. Le précipité est récupéré, lavé à l'éthanol, broyé et séché sous vide. On obtient ainsi 120 g de mélange d'oligosaccharides.

### C - GEL - FILTRATION

60 g du mélange obtenus sont dissous dans 500 ml de NaCl 0,5 M déposés au sommet d'une colonne d'Ultrogel AcA 202®. La colonne est éluée à un débit de 1500 ml /heure par du tampon NaCl 0,5 M.

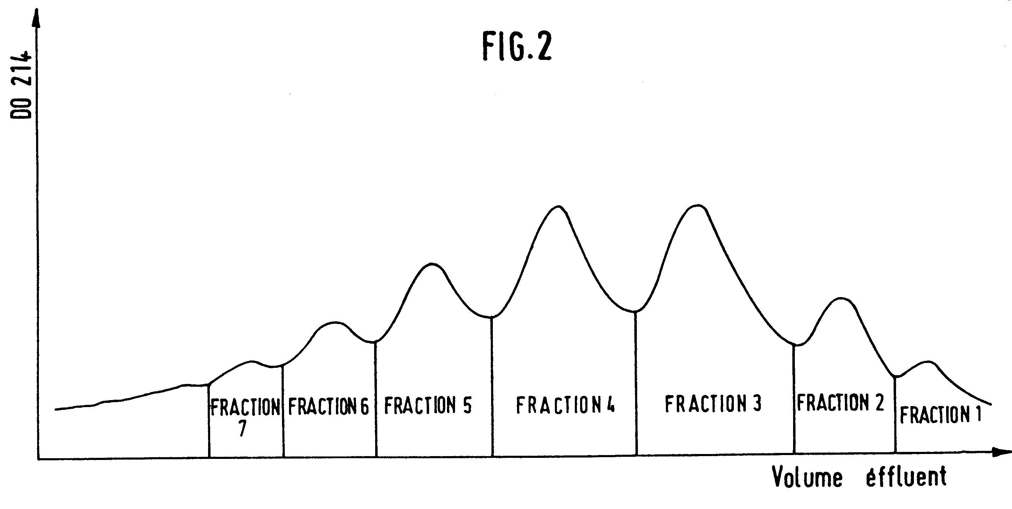

L'absorption UV 214 nm de l'effluent est enregistrée en continu. On obtient le profil d'élution représenté sur la fig. 2. 7 fractions sont séparées et précipitées par 2 volumes d'éthanol.

Ces fractions ont les caractéristiques suivantes :

| | | | | | | | |
|---|---|---|---|---|---|---|---|
| N° fraction | 1 | 2 | 3 | 4 | 5 | 6 | 7 |
| Volume (litre) | 2,1 | 2,2 | 4,1 | 3,9 | 3,3 | 2,5 | 1,8 |
| Intervalles du volume d'élution (litre) | 41-39 | 39-36 | 36-32 | 32-28 | 28-25 | 25-22 | 22-20 |
| Poids produit sec * | 1,2g | 5,6g | 12 g | 9,8g | 8 g | 6,2g | 1,5 g |
| Nature | disaccharide | tétra | hexa | octa | déca | dodéca | tétradéca |

| | | | | | | | |
|---|---|---|---|---|---|---|---|
| * Le produit obtenu par précipitation alcoolique est centrifugé et séché sous vide à 60° C pendant environ 30 heures. | | | | | | | |

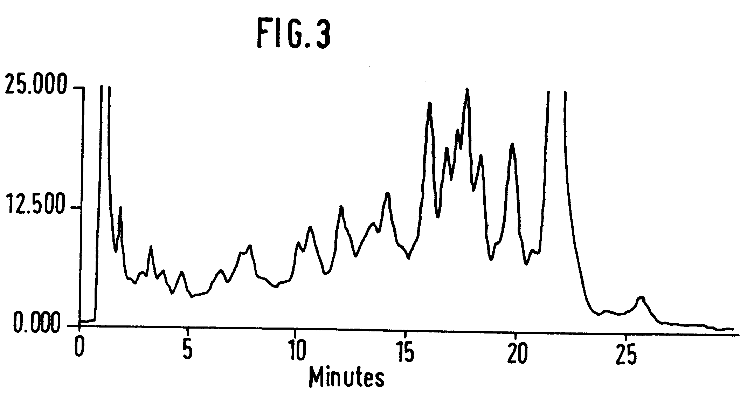

Le profil d'élution en échange d'ions de la fraction hexasaccharidique est représenté sur la figure 3 où l'on indique en abscisses le temps de rétention et en ordonnées les quantités de produits dans l'éluat. Pour effectuer cette mesure, on utilise une colonne Mono-Q® commercialisée par Pharmacia avec un gradient en NaCl de 0,5 à 1,5 M, le débit étant de 1 ml/mn. La détection est effectuée par absorption à 214 nm. L'examen de ce profil d'élution montre l'hétérogénéïté de la fraction.

### D - CHROMATOGRAPHIE D'AFFINITE SUR FGF-SEPHAROSE®

Le FGF anionique est préparé à partir de cervelles de boeuf selon la technique de Roy R. Lobb et James W. Fett, Biochemistry vol.23, N° 26, 1984 pp. 6295-6299.

45 mg de FGF anionique sont fixés sur 30 ml de Sépharose 4 B®. Selon la technique de P.M. Cuatrecasas, M. Wilchek et C.B. Anfinsen, Proc. Nat. Acad. Sci. U.S. 61 (1968), 636.

Les 30 ml de FGF-Sépharose® ainsi obtenus sont placés dans une colonne (2,5 cm x 6,5 cm), et équilibrés en tampon tris-HCl 0,01M pH 7,4, 0,2M NaCl (appelé ci-après tampon I).

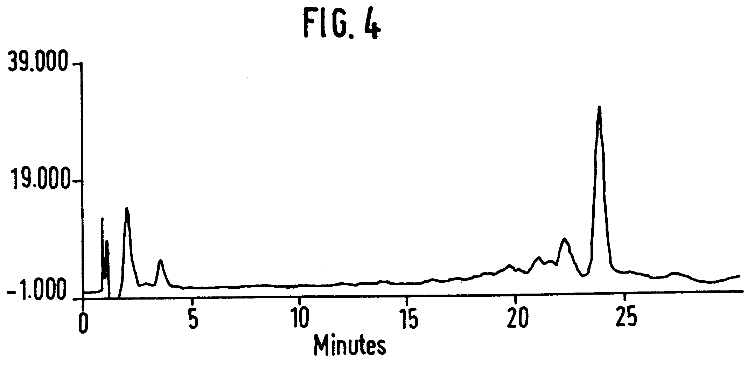

300 mg de la fraction hexasaccharidique en solution dans 60 ml de tampon I sont déposés sur la colonne de FGF-Sepharose®; cette dernière est lavée avec 600 ml de tampon I à un débit de 60 ml par heure. La colonne est finalement éluée par du tampon I ajusté à 1 M NaCl. Les 18 ml d'éluat contenant des substances oligosaccharidiques sont recueillis et précipités par addition de 108 ml d'éthanol pur. On récupère ainsi 0,15 mg d'un hexasaccharide dont le profil d'élution en échange d'ions est représenté sur la figure 4. Ce produit est appelé, dans ce qui suit, IC 1696.

On donne ci-après les caractéristiques du spectre RMN de ¹³C de l'hexasaccharide de la figure 1, les symboles suivants étant utilisés pour représenter les 6 motifs constitutifs : G₁ H₁ G₂ H₂ G₃ AM₃.

La mesure spectrale est réalisée sur une solution de ce produit dissous dans l'eau deutériée, à 25 MHz, référence pour la mesure des déplacements :
TST sel de sodium de l'acide 3-(triméthylsilyl) propionique.

| ppm | Signal | Attribution | Attribution d'une façon plus générale* |
|---|---|---|---|
| 60,5 | 5 | C-2 de H₁+H₂ | C-2 du groupe N-sulfate glucosamine |
| 63,3 | 9 | C-1 de AM₃ | C-1 du groupe anhydromannitol |
| 68,9 | 11 | C-6 de H₁+H₂ | C-6 du groupe glucosamine N-sulfate-6-sulfate |
| 70,7 | 13 | C-6 de AM₃ | C-6 du groupe anhydromannitol 6-sulfate |
| 77,8 | 17 | C-3 de AM₃ | C-3 du groupe anhydromannitol |
| 82,0 | 19 | C-5 de AM₃ | C-5 du groupe anhydromannitol |
| 85,6 | 21 | C-2 de AM₃ | C-2 du groupe anhydromannitol |
| 87,5 | 23 | C-4 de AM₃ | C-4 du groupe anhydromannitol |
| 98,6) | 27 | C-1 de H₁+H₂ | C-1 du groupe glucosamine-N-sulfate |
| 99,2) | | | |
| 102,6) | 29 | C-1 de G₁+G₂ + G₃ | C-1 du groupe iduronique-2-sulfate |
| 101,8) | | | |
| 101,6) | | | |

| | | | |
|---|---|---|---|
| * c'est-à-dire par rapport aux connaissances dans ce domaine sur l'héparine. | | | |

### EXEMPLE 2 :

On procède de manière similaire à l'exemple 1, en utilisant un autre lot d'héparine injectable.

On récupère finalement 115 g de mélange d'oligosaccharides. 60 g de ce mélange sont traités par gel-filtration dans les mêmes conditions que celles décrites dans l'exemple 1.

Les caractéristiques des fractions récupérées sont regroupées dans le tableau ci-dessous :

| | | | | | | | |
|---|---|---|---|---|---|---|---|
| N° fraction | 1 | 2 | 3 | 4 | 5 | 6 | 7 |
| Volume (litre) | 2,2 | 2,2 | 3,1 | 2,0 | 3,4 | 3,1 | 2 |
| Poids produit sec | 4,6 g | 1,9 g | 11,8g | 1,7 g | 10,2g | 7,9 g | 6,0 g |
| Nature | tétra sacchari de | inter médiaire tétra hexa | hexasaccharide | inter médiaire hexaocta | octasaccharide | décasaccharide | dodécasaccha |

300 mg de la fraction 2 (intermédiaire tétra-hexa) sont soumis à la chromatographie d'affinité sur FGF-Sépharose®. On obtient finalement 0,11 mg de produit appelé IC 1697.

300 mg de la fraction hexasaccharidique sont soumis à une chromatographie d'affinité sur FGF-Sépharose ®. On obtient ainsi 0,22 mg d'un produit dont le profil d'élution en gradient d'échange d'ions correspond à celui de la figure 4.

### EXEMPLE 3 :

1,5 g de la fraction hexasaccharidique 3 sont déposés sur une colonne d'échangeur d'anions Q Sépharose® (170 ml, 2,5 cm x 35 cm) équilibrée par du tampon NaCl 0,4M.

La colonne est rincée par du tampon 0,6 M jusgu'à absence de substances oligosaccharidiques dans l'effluent (volume de rinçage 3,5 litres). Les oligosaccharides qui ne sont pas absorbés, récupérés lors du rinçage, correspondent au produit dépourvu d'affinité pour le FGF anionique appelé ci-après P38EXH13.

Elle est alors éluée par du tampon NaCl 1 M, et les 120 ml d'éluat contenant des oligosaccharides sont recueillis et précipités par addition de 720 ml d'éthanol.

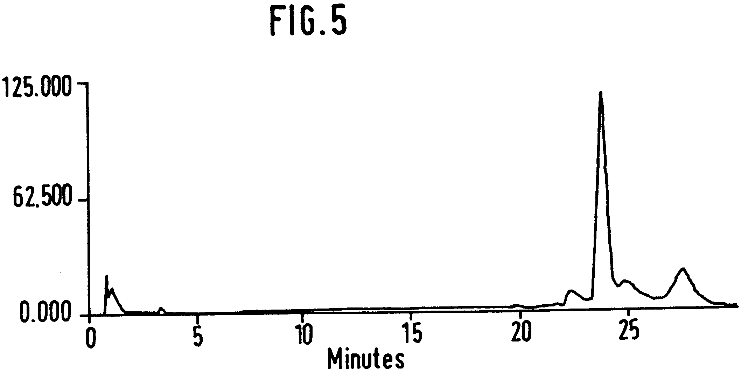

On obtient ainsi 80 mg d'un produit dont le profil d'élution en chromatographie d'échange d'ions est représenté sur la figure 5.

### EXEMPLE 4 :

### - Procédé de séparation d'un hexasaccharide fortement anionique par chromatographie d'affinité sur FGF cationique-Sépharose® :

La préparation de FGF cationique de cervelle de boeuf et la fixation du FGF cationique sur la Sépharose® s'effectuant selon les mêmes techniques que celles décrites pour le FGF anionique.

Une colonne (1,6 cm x 5 cm) contenant 10 ml de FGF cationique-Sépharose® (1,5 mg de FGF cationique par ml de Sépharose) est équilibrée en tampon I.

100 mg d'hexasaccharide (obtenu selon l'exemple 1) en solution dans 20 ml de tampon I sont déposés sur la colonne. La colonne est lavée à l'aide de 200 ml de tampon I, puis éluée par du tampon I ajusté à 2 M NaCl.

Les 10 ml d'éluat contenant les substances oligosaccharidiques sont recueillis et précipités par addition de 50 ml d'éthanol pur.

On récupère ainsi 0,050 mg d'un hexasaccharide dont le profil d'élution en échange d'ions correspond à celui de la figure 4.

### EXEMPLE 5 :

### - Variantes de préparation d'un mélange de dépolymérisation renfermant les oligosaccharides de l'invention :

### . Préparation des oligosaccharides par dégradation periodique :

10 g d'héparine sont mis en solution dans 200 ml d'eau déminéralisée.

On ajoute du métaperiodate de sodium en quatité suffisante pour avoir une molarité finale de 0,25 M soit 10,7 g, et le pH de la solution est abaissé à 3,0 à l'aide d'HCl 5 N.

La solution est laissée au repos, à 25°C, à l'obscurité pendant 48 heures.

Le pH est amené à 7 à l'aide de soude 5N, et l'on ajoute 300 ml d'éthanol pur. Le précipité formé est récupéré, broyé, lavé à l'éthanol et à l'acétone, puis finalement séché sous vide à 60° C.

Il est redissous dans 120 ml de soude 0,2 N contenant 2 mg de borohydrure de sodium par ml. La solution est agitée 15 heures à température ambiante. Le pH est ajusté à 4 à l'aide d'HCl 5 N puis de nouveau à 7 par de la soude 5 N. On ajoute 200 ml d'éthanol pur.

Le précipité formé est centrifugé, broyé, lavé à l'éthanol et à l'acétone et séché sous vide à 60° C.

On obtient ainsi 6,4 g d'un mélange d'oligosaccharides. Ce mélange est soumis à l'étape de gel-filtration sur Ultrogel AcA 202® dans les conditions de l'exemple 1.

### . Préparation des oligosaccharides par action enzymatique de l'héparinase ou de l'héparitinase :

L'héparinase ou l'héparatinase sont extraites de Flavobacterium Heparinum et purifiées selon la technique décrite par P. HOVINGH et A. LINKER, J. Biol. Chem. vol. 245 n° 22, 1970, pp. 6170-6175.

### - Dépolymérisation par l'héparinase :

10 g d'héparine sont mis en solution dans 500ml de tampon acétate 0,1 M pH 7,0, CaCl₂ 0,01 M.

La solution est mise au bain-marie à 30°C et l'on ajoute 2,5 mg d'héparinase pure.

On laisse incuber 24 heures à 30°C. On ajoute 1200 ml d'éthanol pur.

Le précipité formé est récupéré par centrifugation, lavé à l'éthanol et séché sous vide à 60°C.

On obtient 7,1 g d'un mélange d'oligosaccharides qui est ultérieurement soumis à la gel-filtration sur Ultrogel AcA 202® dans les conditions de l'exemple 1.

### - Dépolymérisation par l'héparitinase :

10 g d'héparine sont mis en solution dans 500 ml de tampon acétate 0,1 M pH 7,0, CaCl₂ 0,01 M.

La solution est mise au bain-marie à 40°C et l'on ajoute 5 mg d'héparitinase pure. On incube à 40°C pendant 10 heures, puis on ajoute 3 mg d'héparitinase pure. On incube de nouveau 6 heures et l'on rajoute de nouveau 3 mg d'héparitinase. 10 heures après ce dernier ajout, on précipite les produits de la réaction par addition de 1200 ml d'éthanol.

Le précipité formé est recueilli par centrifugation, lavé à l'éthanol et séché sous vide à 60° C.

On obtient 6,8 g d'un mélange d'oligosaccharides qui est ultérieurement soumis à la gel-filtration sur Ultrogel AcA 202® dans les conditions de l'exemple 1.

### EXEMPLE 6 :

### - Préparation d'octasaccharides à haute affinité pour le FGF anionique par chromatographie d'affinité sur FGF-Sépharose®.

La colonne de chromatographie est préparée comme décrit à l'exemple 1 D.

300 ml de la fraction octasaccharidique telle qu'obtenue à l'exemple 1 en solution dans 60 ml de tampon I sont déposés sur la colonne de FGF-Sépharose®; cette dernière est lavée avec 600 ml de tampon I à un débit de 60 ml par heure. La colonne est finalement éluée par du tampon I ajusté à 1 M NaCl.

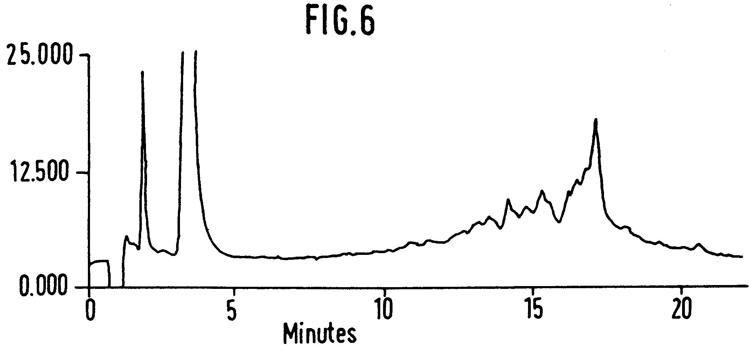

Les 20 ml d'éluat contenant des substances oligosaccharidiques sont recueillis et précipités par addition de 120 ml d'éthanol pur. On récupère ainsi 0,26 mg d'un mélange d'octasaccharides ayant le profil d'élution en échange d'ions représenté sur la figure 6.

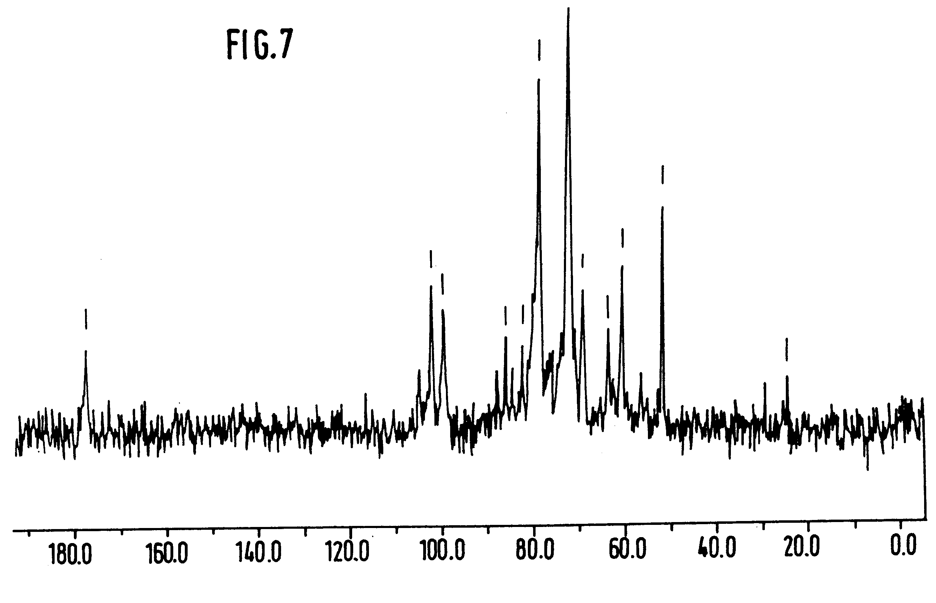

Le spectre RMN du ¹³C dans l'eau deutériée, à 25 MHz, pour le mélange d'octosaccharides obtenu, est donné sur la figure 7.

### EXEMPLE 7 :

### - Préparation de décasaccharides à haute affinité pour FGF anionique par chromatographie d'affinité sur FGF-Sépharose®.

La colonne de chromatographie est préparée comme décrit à l'exemple 1 D.

300 mg de la fraction décasaccharidique telle qu'obtenue à l'exemple 1 en solution dans 60 ml de tampon I sont déposés sur la colonne de FGF-Sépharose®; cette dernière est lavée avec 600 ml de tampon I à un débit de 60 ml par heure. La colonne est finalement éluée par du tampon I ajusté à 1 M NaCl.

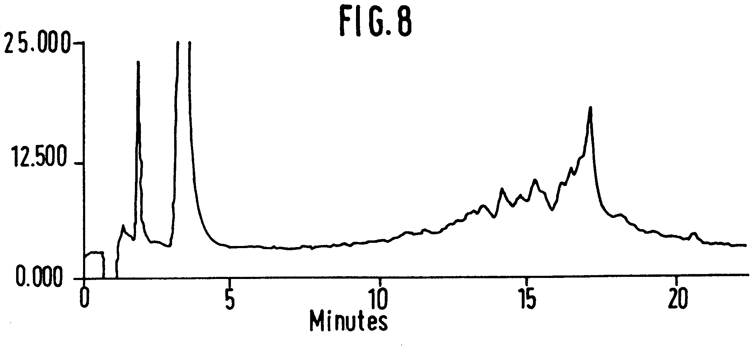

Les 26 ml d'éluat contenant des substances oligosaccharidiques sont recueillis par addition de 160 ml d'éthanol pur. On récupère ainsi 0,38 mg d'un mélange de décasaccharides ayant le profil d'élution en échange d'ions représenté sur la figure 8.

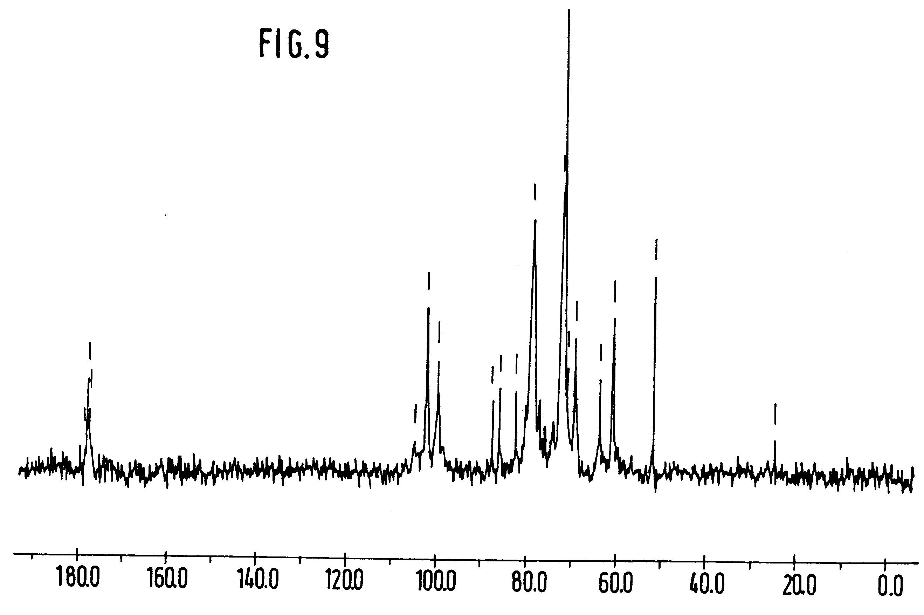

Le spectre RMN du ¹³C dans l'eau deutériée, à 25 MHz, pour le mélange de décasaccharides obtenu, est donné à la figure 9.

### EXEMPLE 8 :

### - Obtention d'un mélange de fragments de glycosaminoglycanes affines pour le FGF anionique :

On opère comme dans l'exemple 1, mais sans effectuer l'étape de gel filtration C.

La chromatographie d'affinité sur FGF-Sépharose ®est alors effectuée sur un mélange renfermant les di-, tétra-, hexa-, octa-, déca-, dodéca- et tétradécasaccharides du tableau de la page 21 .

Cette opération de chromatographie permet d'isoler celles des chaïnes du mélange qui sont dotées d'une affinité pour le FGF.

### EXEMPLE 9 :

On prépare 120 mg d'hexasaccharide selon la technique décrite dans l'exemple 3 ci-dessus, en omettant la phase de réduction au térahydruroborate de sodium lors de la dépolymérisation initiale de l'héparine selon l'exemple 1.

Ces 120 mg d'hexasaccharide sont mis en solution dans 100 ml de NaCl 0,5 M. On ajoute 50 ml de Sépharose ®-aminoéthyle (contenant environ 8 µmoles de groupe aminoéthyle par ml de matrice décantée) et 50 mg de cyanoborohydrure de sodium. L'ensemble est porté à pH 11 à l'aide de soude 5 N. et maintenu sous agitation modérée, à température ambiante, pendant 10 jours.

La matrice est ensuite filtrée sur büchner, lavée par 1 litre de solution de NaCl 0,5 M pH 7, puis par 1 litre d'acide chlorhydrique 10⁻³N, puis par 500 ml de NaCl 1 M pH 10,0, puis finalement par 1 litre d'eau distillée.

Un aliquote de 5 ml de matrice est prélevé et hydrolysé 3 heures à 100° C dans l'acide chlorhydrique 2 M. Le dosage des acides uroniques dans l'hydrolysat montre que la matrice finalement obtenue contient 2 mg d'hexasaccharide lié de manière covalente par ml de Sépharose®.

40 ml de matrice Sépharose®-hexasaccharide sont placés dans une colonne de diamètre 2,5 cm et de hauteur 8 cm.

La colonne est équilibrée par du tampon tris-HCl 0,02 M pH 7,0, 0,3 M NaCl, (tampon 1).

2 mg de FGF anionique de cervelle de boeuf (préparé selon la technique de R.R. LOBB et J.W. FETT, Biochemistry, vol. 23, n° 26, 1984, p. 6295-6299), en solution dans 10 ml de tampon I sont percolés à travers la colonne qui est ensuite rincée à l'aide de 120 ml de ce même tampon I.

La colonne est alors éluée par un gradient linéaire obtenu par le mélange progressif de 200 ml de tampon I et de 200 ml de tampon tris-HCl 0,02 M pH 7,0, 2 M NaCl.

Le pic de FGF anionique émerge de la colonne à une force ionique d'environ 0,7 M, et est récupéré avec un rendement de 95%.

### EXEMPLE 10 :

10 mg de décasaccharide sont préparés selon la technique décrite dans l'exemple 7 ci-dessus en omettant la phase de réduction au tétrahydruroborate lors de la dépolymérisation initiale de l'héparine selon l'exemple 1.

Ces 10 mg de décasaccharide sont mis en solution dans 20 ml d'eau distillée. On ajoute 5 ml de Sépharose ®-aminoéthyle (contenant 8 µmoles de groupe aminoéthyle par ml) et 5 mg de cyanoborhydrure de sodium. le pH de la suspension est ajusté à pH 11 à l'aide de soude 5 N et maintenu sous agitation modérée, à température ambiante pendant une semaine.

La matrice de Sépharose® est ensuite filtrée sur büchner, lavée par 100 ml de solution NaCl 0,5 M pH 7, puis par 100 ml d'acide chlorhydrique 10⁻³ N puis par 100 ml de NaCl 2 M et enfin par 200 ml d'eau distillée.

1 ml de matrice est prélevé et hydrolysé 3 heures dans 5 ml d'acide chlorhydrique 2 M. Le dosage des acides uroniques dans l'hydrolysat montre que la matrice obtenue contient 1,8 mg de décasaccharide fixé de manière covalente par ml de Sépharose®.

4 ml de Sépharose®-décasaccharide sont placés dans une colonne de diamètre 1 cm et de hauteur 5 cm. La colonne est équilibrée par du tampon tris-HCl 0,02 M pH 7,0, 0,6 M NaCl (tampon II). 0,2 mg de FGF cationique de cervelle de boeuf préparé par la même technique que celle citée dans l'exemple 1 en solution dans 2 ml de tampon II est déposé au sommet de la colonne. La colonne est rincée à l'aide de 40 ml de tampon II. Elle est finalement éluée par du tampon tris-HCl 0,02 M pH 7,0, 2 M NaCl. Le FGF anionique est élué sous forme de pic étroit. La quantité retrouvée dans l'éluat est de 0,18 mg correspondant à un rendement de 90% .

### EXEMPLE 11 :

Selon une variante de réalisation de l'exemple 1, l'étape de dépolymérisation est effectuée par oxydation periodique en procédant comme suit :

### 1/ - Coupure des chaînes d'héparine à l'aide d'acide periodique

10 g d'héparine injectable de mucus de proc, sous forme de sel de sodium, titrant 157 uI/mg dans le dosage Codex et 155 u/mg dans le dosage anti-facteur Xa de Yin et al; sont mis en solution dans 250 ml d'eau déminéralisée à 4° C. Le pH de la solution est ajusté à 5,0 par de l'acide chlorhydrique concentré. 10 g de métaperiodate de sodium (NaIO₄, PM : 213,89) en solution dans 250 ml d'eau déminéralisée à 4° C sont ajoutés sous agitation modérée. Le pH de l'ensemble est ajusté à 5,0 par de l'acide chlorhydrique concentré. La solution est abandonnée 24 heures, à l'obscurité, en chambre froide à + 4° C.

### 2/ - Elimination du periodate résiduel

On répartit ensuite la solution réactionnelle dans 3 boyaux de dialyse NOJAX 40®, (porosité de 3 à 4000 Da) et on la soumet à une dialyse 15 heures contre de l'eau déminéralisée courante.

### 3/ - Dépolymérisation en milieu basique

Aux 780 ml de solution obtenue après dialyse, sont ajoutés 16 ml de soude 10 N, et l'ensemble est soumis à agitation 3 heures à température ambiante (de l'ordre de 18-21° C).

Le cas échéant, cette étape est suivie d'une réduction réalisée comme suit :
500 mg de borohydrure de sodium (NaBH₄, PM : 37,83) sont alors ajoutés et la solution est agitée de nouveau 4 heures à température ambiante. Son pH est ensuite amené à 4 à l'aide d'acide chlorhydrique concentré. Après 15 minutes d'agitation, le pH est ajusté à 7 à l'aide de soude concentrée.

Aux 820 ml de solution ainsi obtenue sont ajoutés 16,4 g de NaCl puis 1270 ml d'éthanol.

L'ensemble est laissé au repos 3 heures, puis centrifugé à 2500 t/minute pendant 20 minutes.

Le précipité est recueilli, remis en suspension dans 200 ml d'ethanol pur, broyé à l'Ultra-Turrax® et finalement récupéré par filtration sur bèchner fritté.

Il est alors séché sous vide à 40° C pendant 5 heures.

On récupère ainsi 8,9 g de produit intermédiaire ayant les propriétés suivantes :

| | |
|---|---|
| - dosage Codex | 8 uI/mg |
| - dosage APTT | 7 uI/mg |
| - dosage AntiXa | 8 u/mg |

Le mélange de chaînes obtenu est soumis à un gel filtration comme décrit dans l'exemple 1.

### EXEMPLE 12 :

### - Toxicité :

IC 1696, 1701 et 1702 ont été administrés à des souris pendant une durée de 15 jours par voies intraveineuse et sous-cutanée et n'ont montré aucune toxicité. Leur DL 50 dans les deux cas est supérieure à 2 g/kg.

### EXEMPLE 13 :

### - Activité des produits de l'invention sur un modèle de culture cellulaire.

### A - DESCRIPTION DU MODELE :

Des cellules LE II (endothéliales de poumon de souris) sont cultivées dans des plaques de 24 puits en présence de 10% de sérum de veau foetal jusqu'au 3/4 de la confluence.

Elles sont ensuite arrêtées 48 heures en 0,2% seulement de sérum et rendues quiescentes.

On ajoute ensuite au milieu soit le "fibroblast growth factor" (FGF), soit les fragments oligosaccharidiques à tester, soit les deux produits simultanément (FGF + produit à tester).

La culture est encore maintenue pendant 24 heures et de la thymidine tritiée est ajoutée 4 heures avant la fin de l'expérience.

La radioactivité incorporée est ensuite comptée; chaque mesure est faite en triplicate.

### B - RESULTATS

Ils sont donnés dans les figures suivantes :

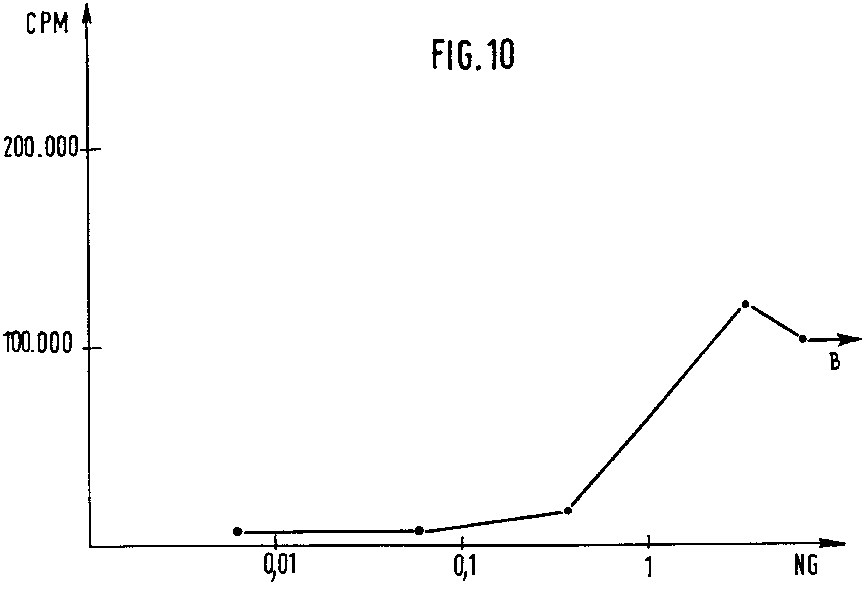

- Figure 10, courbe dose-réponse en présence du FGF anionique seul.

Le plateau de stimulation se situe à 126.000 cpm pour une dose de 5 ng/ml. Dans les figures suivantes, la stimulation maximale en présence de FGF anionique seul est représentée par la ligne en pointillés, les courbes △_{―}△ représentant les résultats obtenus lorsque le glycosaminoglycane est utilisé seul et les courbes ._{―}. ceux obtenus lorsque le glycosaminoglycane est utilisé avec du FGF anionique.

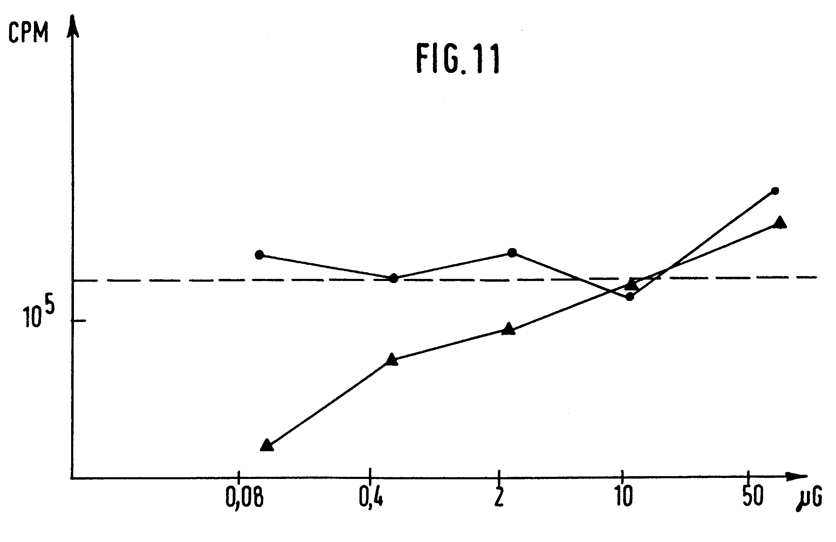

- Figure 11, courbe dose-réponse en présence de l'héxasaccharide IC 1696, seul ou avec du FGF anionique (5 ng/ml).

On constate que IC 1696 a une activité dose-dépendante de 0,08 à 50 µg/ml de milieu de culture.

Associé au FGF anionique, on constate une augmentation par rapport à l'activité du FGF seul, à partir de 10 µg/ml.

Dans les conditions expérimentales et dans ce système cellulaire, IC 1696 potentialise l'activité du FGF anionique.

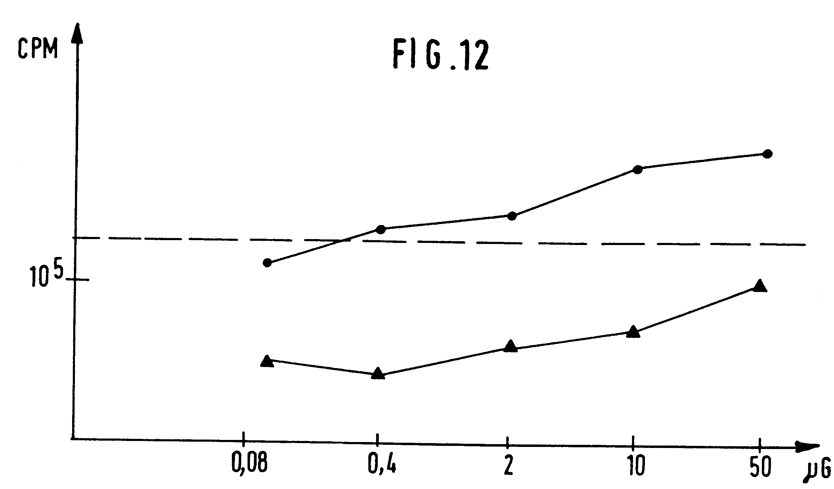

- Figure 12, courbe dose-réponse en présence de l'hexasaccharide IC 1697, seul ou avec du FGF anionique (5 ng/ml).

IC 1697 utilisé seul montre une activité dose-dépendante entre 0,04 et 50 µg/ml.

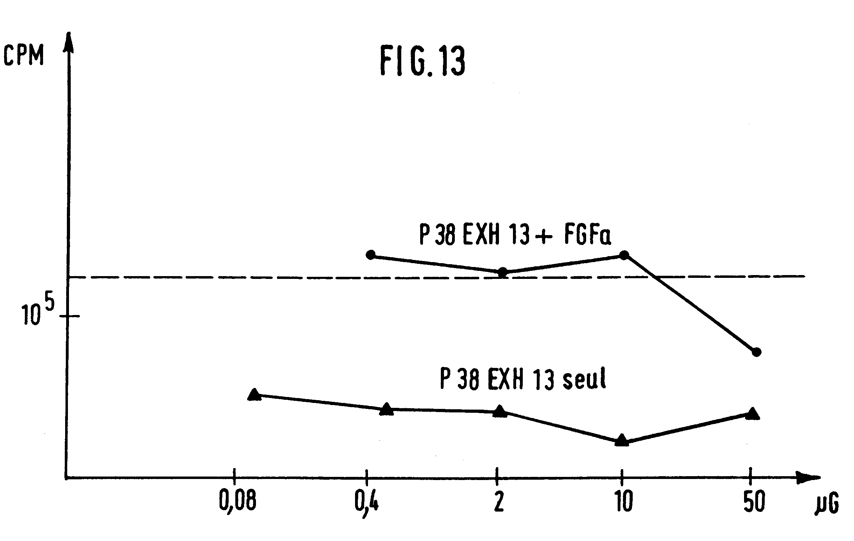

Utilisé en association avec le FGF anionique, dans les conditions expérimentales du modèle, il est nettement potentialisateur à partir de 0,4 µg/ml. - Figure 13, courbe dose-réponse en présence d'un produit de contrôle dépourvu d'affinité pour le FGF anionique : l'hexasaccharide p 38 EXH 13, seul ou avec du FGF anionique (5 ng/ml).

Le fragment utilisé comme référence ne montre pas d'activité significative lorsqu'il est utilisé seul.

En conclusion, dans ce modèle expérimental (cellules quiescentes en absence de sérum), l'effet des oligosaccharides ayant de l'affinité pour le FGF est un effet stimulateur (mitogénique).

### EXEMPLE 14 :

### - Activité des oligosaccharides sur la prolifération de cellules endothéliales aortiques bovines en présence de FGF basique :

Des cellules endothéliales aortiques bovines sont ensemencées à basse densité (20.000 cellules par boïte de 35 mm) dans du milieu Eagle modifié contenant 10% de sérum de veau foetal.

Les cellules sont maintenues 4 jours en culture et additionnées au jour J.O. et J+2 de 10 µl d'une solution de FGF basique à la concentration de 8,6 pg/µl.

De plus, au jour J.O., les cultures sont additionnées ou non des oligosaccharides à tester à des concentrations variées. Au bout de 4 jours, on effectue le comptage des cellules à l'aide d'un compteur de particules Coulter.

Les résultats sont exprimés en pourcentage d'inhibition de la prolifération des cellules par comparaison avec des cultures n'ayant pas reçu les produits à tester. A titre de comparaison, des essais sont également effectués avec de l'héparine.

Les résultats sont les suivants :
Ils sont exprimés en DE 50, c'est-à-dire à quantité de produit qui est nécessaire pour inhiber de 50% la prolifération cellulaire obtenue dans les cellules ne contenant que le FGF :

| Produits testé | DE 50 |
|---|---|
| IC 1696 | 1. µg/ml |
| IC 1701 | 1,8 µg/ml |
| IC 1702 | 40. µg/ml |
| Héparine | 1. µg/ml |

A titre de comparaison, on a également testé l'activité de tétrasaccharides et disaccharides réguliers obtenus par gel filtration et chromatographie d'échange d'ions après dépolymérisation à l'acide nitreux. On a pu constater que ces produits, dépourvus d'affinité pour le FGF, n'avaient aucune activité inhibitrice dans le modèle utilisé.

En revanche, IC 1696 et IC 1701 ont sensiblement la même activité que l'héparine et IC 1702 est encore très fortement actif.

### EXEMPLE 15 :

### - Effet des oligosaccharides sur la prolifération des cellules endothéliales humaines en culture.

Des cultures primaires de cellules endothéliales de la veine ombilicale sont préparées d'après le procédé de Jaffe (J. Clin. Invest., 1973, 52, 2745). Au moment de la confluence (5-7 jours), les cellules sont décollées par un mélange trypsine-EDTA. Elles sont ensuite repiquées sur des plaques Falcon de 24 puits, préalablement recouvertes par de la fibronectine (5 µg/cm²), à une densité de 5.000 cellules/cm² dans du milieu 199 additionné de 20% de sérum de veau foetal (SVF).

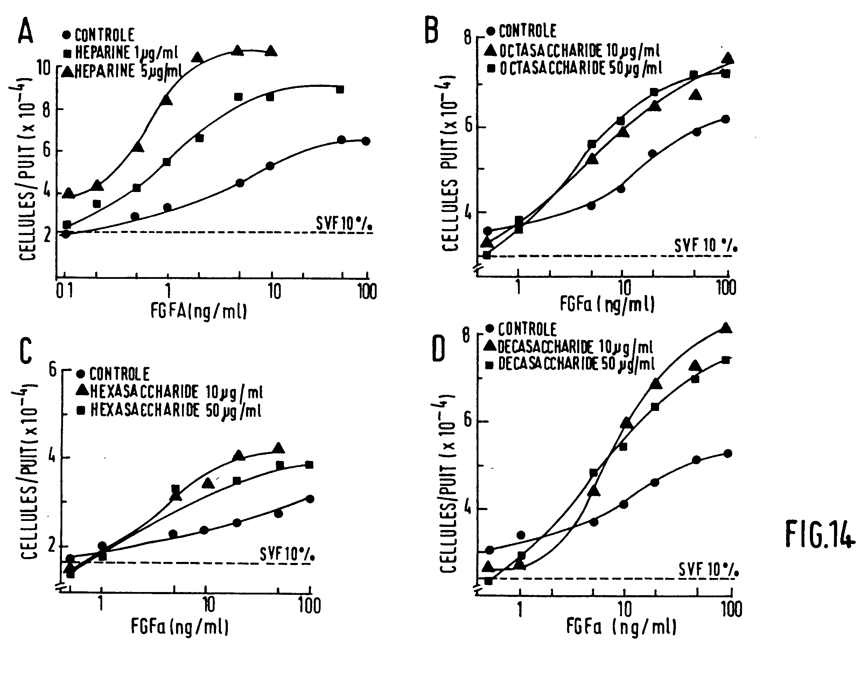

Vingt-quatre heures après, le milieu est remplacé par du milieu 199 additionné de 10% de SVF et l'on ajoute les glycosaminoglycanes seuls ou en association avec ce FGF acide (FGFa). 48 heures après, on ajoute à nouveau le facteur de croissance. Au bout de 4 jours de culture, les cellules sont décollées par le mélange trypsine-EDTA et leur comptage est effectué à l'aide d'un compteur de cellules (Coulter Counter®Coultronics). Chaque courbe expérimentale (Fig. 14-A-D) représente une expérience type conduite en triplicate. Chaque expérience est répétée 2-3 fois.

Sur les figures 14 A-D, la ligne en pointillé représente la prolifération basale pour une culture contenant 10% de SVF sans FGFa et sans oligosaccharide; la courbe avec ._{―}._{―}. représente l'essai contrôle en présence de FGFa seul; les courbes ■_{―}■_{―}■ et ▲_{―}▲_{―}▲ représentent les résultats obtenus en présence de FGFa associé à des concentrations de 1 µg/ml et 5 µg/ml d'héparine, ou de 10 µg/ml et 50 µg/ml d'oligosaccharides respectivement.

Dans nos conditions expérimentales, le FGFa sans association aux oligosaccharides provoque une augmentation maximale de 230% du nombre de cellules par rapport au SVF seul à la concentration de 10%, la DE 50 est de 7 ng/ml. On entend par DE 50 la concentration de FGFa (ng/ml) qui a déterminé 50 % de prolifération, par rapport à la prolifération maximale, obtenue avec 50 - 100 ng/ml de FGFa.

### RESULTATS

Les effets des oligosaccharides ont été examinés en comparaison avec l'héparine standard de départ (lot 91416).

Dans les conditions expérimentales choisies, l'héparine potentialise l'effet du FGFa. Cet effet potentialisateur commence à une concentration en héparine de 200 ng/ml et atteint son maximum à une concentration en héparine de 25 µg/ml. Une concentration en héparine de 1 µg/ml diminue la DE 50 du FGFa de dix fois (Fig. 14A). On constate que les oligosaccharides testés ont des effets différents selon les conditions expérimentales mises en oeuvre.
1/ - En absence de FGFa, à la concentration de 1 à 100 ug/ml, les oligosaccharides inhibent la croissance des cellules endothéliales (Tableau ci-après).

| EFFET INHIBITEUR DE GLYCOSAMINOGLYCANES SUR LA CROISSANCE DES CELLULES ENDOTHELIALES HUMAINES EN PRESENCE DE SVF ET EN L'ABSENCE DE FGFa | | | |
|---|---|---|---|
| | INHIBITION (%) | | |
| | 1 µg/ml | 10 µg/ml | 100 µg/ml |
| Hexasaccharide IC 1696 | 8,4 | 25,5 | 34,9 |
| Octosaccharide IC 1701 | 5,9 | 25,4 | 19,5 |
| Décasaccharide IC 1702 | 11,0 | 22,4 | 10,0 |

2/ - A des concentrations de FGFa supérieures à la DE 50 (7 ng/ml), on constate un effet stimulateur des oligosaccharides sur la croissance cellulaire (Fig. 14 B-D). Cet effet nécessite des concentrations plus fortes d'oligosaccharides que d'héparine. Le produit le plus actif est le décasaccharide IC 1702 (à 70 µg/ml, la DE 50 s'abaisse à 3,2 µg/ml). L'hexasaccharide IC 1696 a un effet de stimulation de la croissance cellulaire très faible. La propriété de potentialisation du FGFa est en bonne corrélation avec l'affinité de ces oligosaccharides pour le facteur de croissance.

En conclusion, le test utilisé met en évidence deux effets des oligosaccharides : un effet inhibiteur en présence de sérum et en absence de FGFa, et un effet stimulateur en présence du facteur de croissance pour lequel ils ont une affinité. Ceci suggère un rôle modulateur des glycosaminoglycanes sur la croissance cellulaire. L'effet inhibiteur permet d'envisager une action antiangiogénèse *in vivo*. L'effet stimulateur, en association avec le facteur de croissance, permet d'envisager l'utilisation de ces produits soit pour la stabilisation et la potentialisation du facteur de croissance, soit pour stimuler la réparation tissulaire.

L'activité de l'hexasaccharide IC 1696 a été testée sur un modèle d'angiogénèse adapté de J. Folkman et al. (Science, 1983, 221, 719), les implants de polymère imprégnés étant remplacés par des injections intraveineuse du produit à tester.

Les lapins reçoivent quotidiennement par voie intraveineuse deux injections de 10 mg/kg de l'hexasaccharide IC 1696 en solution dans du soluté chloruré isotonique et en association ou non avec des stéroïdes.

On constate chez les animaux traités par IC 1696 une vascularisation nettement moins importante chez les animaux ayant reçu simplement le soluté chloruré isotonique; en particulier, chez les premiers, la vascularisation secondaire est beaucoup moins développée.

Dans un essai préliminaire utilisant le même modèle, IC 1696 seul a montré une action inhibitrice de l'angiogénèse.

## Revendications (Revendications pour l'(les) Etat(s) contractant(s) suivant(s): BE, CH, DE, FR, GB, GR, IT, LI, LU, NL, SE)

1. Oligosaccharides, caractérisés en ce qu'ils répondent à la formule III suivante :
R(GH)ₙR' III
dans laquelle
- n est un nombre de 2 à 6,
- G-H correspond à un enchaînement disaccharidique de structure (acide L-iduronique 2-O-sulfate)-(D-glucosamine NH-sulfate 6-O-sulfate),
- G étant un radical d'acide L-iduronique 2-O-sulfate,
- H étant un radical de D-glucosamine NH-sulfate 6-O-sulfate,
- R représente soit un atome d'hydrogène, soit une unité dissacharidique G'-H dans lequel le radical G' est un radical d'acide uronique α,β insaturé,
- R' représente soit un atome d'hydrogène, soit une unité disaccharidique G-H' dans lequel le radical H' est un radical de 2,5-anhydromannitol ou d'acide 2,5-anhydromannonique,
étant entendu que les significations de R et R' sont telles que le nombre total d'unités monosaccharidiques des oligosaccharides, y compris les unités modifiées, est au plus égal à 14,
- capables de se fixer à FGF anionique, fixé sur une colonne de chromatographie, en milieu tampon tris-HCl 0,01 M pH 7,4, 0,2 M NaCl,
et leurs sels pharmacologiquement acceptables.

2. Oligosaccharide selon la revendication 1, caractérisé en ce qu'il est formé de chaînes renfermant 6 unités monosaccharidiques.

3. Hexasaccharide selon la revendication 2, caractérisé en ce qu'il correspond :
- à un produit contenu dans une fraction correspondant à celle éluée entre un volume de 32 à 36 l dans un système de gel filtration où l'on dépose, en solution dans 500 ml de NaCl 0,5 M, 60 g de précipité, obtenu par précipitation alcoolique d'un mélange de dépolymérisation nitreuse de l'héparine, sur une colonne de 100 cm x 25 cm contenant 45 litres d'agarose-acrylamide, équilibrée avec un tampon de NaCl 0,5 N, pH 6,0 et où l'on effectue l'élution à un débit de 1500 ml/heure,
- ce produit étant séparé par chromatographie d'affinité sur FGF anionique fixé sur support d'agarose, en utilisant une colonne de 2,5 cm x 6,5 cm, équilibrée en tampon tris-HCl 0,01 M, pH 7,4, 0,2 M NaCl renfermant 30 ml de FGF anionique fixé sur support d'agarose, sur laquelle on dépose 300 mg de la frac-tion ci-dessus en solution dans 60 ml du même tampon, en éluant à l'aide de ce tampon ajusté à 1 M de NaCl et en précipitant l'oligosaccharide de la fraction recueillie à l'aide d'éthanol.

4. Oligosaccharides selon la revendication 1, caractérisés en ce qu'ils sont formés de chaînes renfermant 8 unités monosaccharidiques.

5. Octosaccharide selon la revendication 4, caractérisé en ce qu'il correspond :
- à un produit contenu dans une fraction correspondant à celle éluée entre un volume de 28 à 32 l dans un système de gel filtration où l'on dépose, en solution dans 500 ml de NaCl 0,5 M, 60 g de précipité, obtenu par précipitation alcoolique, d'un mélange de dépolymérisation nitreuse de l'héparine, sur une colonne de 100 cm x 25 cm contenant 45 litres d'agarose-acrylamide, équilibrée avec un tampon de NaCl 0,5 N, pH 6,0 et où l'on effectue l'élution à un débit de 1500 ml/heure,
- ce produit étant séparé par chromatographie d'affinité sur FGF anionique fixé sur support d'agarose, en utilisant une colonne de 2,5 cm x 6,5 cm, équilibrée en tampon tris-HCl 0,01 M, pH 7,4, 0,2 M NaCl renfermant 30 ml de FGF anionique sur support d'agarose, sur laquelle on dépose 300 mg de la fraction ci-dessus en solution dans 60 ml du même tampon, en éluant à l'aide de ce tampon ajusté à 1 M de NaCl et en précipitant l'oligosaccharide de la fraction recueillie à l'aide d'éthanol.

6. Oligosaccharides selon la revendication 1, caractérisés en ce qu'ils sont formés de chaînes renfermant 10 unités monosaccharidiques.

7. Decasaccharide selon la revendication 6, caractérisé en ce qu'il correspond :
- à un produit contenu dans une fraction correspondant à un volume d'élution entre 25 et 28 litres dans un système de gel filtration où l'on dépose en solution dans 500 ml de NaCl 0,5 M, 60 g de précipité obtenu par précipitation alcoolique d'un mélange de dépolymérisation nitreuse de l'héparine, sur une colonne de 100 cm x 25 cm contenant 45 litres d'agarose-acrylamide, équilibrée avec un tampon de NaCl 0,5 M pH 6,0, et où l'on effectue l'élution à un débit de 1500 ml/heure,
- ce produit étant ensuite séparé par chromatographie d'affinité sur FGF anionique sur support d'agarose, en utilisant une colonne de 2,5 cm x 6,5 cm, équilibrée en tampon tris-HCl 0,01 M pH 7,4, 0,2 M NaCl renfermant 30 ml de FGF anionique fixé sur support d'agarose, sur laquelle on dépose 300 mg de la fraction ci-dessus, en solution dans 60 ml du même tampon, en éluant à l'aide de ce tampon ajusté à 1 M de NaCl et en précipitant l'oligosaccharide de la fraction recueillie à l'aide d'éthanol.

8. Oligosaccharides selon la revendication 1, caractérisés en ce qu'ils sont formés de chaînes renfermant 12 unités monosaccharidiques.

9. Procédé d'obtention d'oligosaccharides selon l'une quelconque des revendications 1, 2, 4, 6 et 8, caractérisé en ce qu'on dépose une préparation de glycosaminoglycanes d'héparine au sommet d'une colonne de chromatographie renfermant le facteur de croissance anionique ou cationique fixé à un support d'agarose, cette colonne étant équilibrée à l'aide d'un tampon de force ionique correspondant à une concentration de 0,2 M de NaCl, et en ce qu'on élue la fraction oligosaccharidique retenue sur la colonne en ajustant le tampon à une force ionique correspondant à une concentration de 1 M à 2 M de NaCl.

10. Procédé d'obtention d'oligosaccharides selon l'une quelconque des revendications 1, 2, 4, 6 et 8, caractérisé en ce qu'on soumet une préparation de glycosaminoglycanes d'héparine à une chromatographie d'échange d'ions sur une colonne échangeuse d'anions basiques forts tels que des ammoniums quaternaires, on fait varier la force ionique du tampon d'élution selon un gradient correspondant à une variation de force ionique d'un tampon de NaCl de 0,5 ± 0,1 M à 1-2 M, et on récupère les fractions les plus anioniques.

11. Compositions pharmaceutiques, caractérisées en ce qu'elles renferment dans leur principe actif au moins un oligosaccharide selon l'une quelconque des revendications 1 à 8 en association avec un véhicule pharmaceutique.

12. Agent modulateur de la croissance de cellules en culture, caractérisé en ce qu'il renferme un oligosaccharide selon l'une quelconque des revendications 1 à 8.

13. Réactif biologique, caractérisé en ce qu'il est élaboré à partir d'un oligosaccharide selon l'une quelconque des revendications 1 à 8.

## Revendications (Revendications pour l'(les) Etat(s) contractant(s) suivant(s): AT, ES)

1. Procédé d'obtention d'oligosaccharides répondant à la formule III suivante :
R(GH)ₙR' (III)
dans laquelle
- n est un nombre de 2 à 6,
- G-H correspond à un enchaînement disaccharidique de structure (acide L-iduronique 2-O-sulfate)-(D-glucosamine NH-sulfate 6-O-sulfate),
- G étant un radical d'acide L-iduronique 2-O-sulfate,
- H étant un radical de D-glucosamine NH-sulfate 6-O-sulfate,
- R représente soit un atome d'hydrogène, soit une unité disaccharidique G'-H dans lequel le radical G' est un radical d'acide uronique α-β insaturé,
- R' représente soit un atome d'hydrogène, soit une unité disaccharidique G-H' dans lequel le radical H' est un radical de 2,5-anhydromannitol ou d'acide 2,5-anhydromannonique,
étant entendu que les significations de R et R' sont telles que le nombre total d'unités monosaccharidiques des oligosaccharides, y compris les unités modifiées, est au plus égal à 14,
- capables de se fixer à FGF anionique, fixé sur une colonne de chromatographie, en milieu tampon tris-HCl de 0,01 M pH 7,4, 0,2 M NaCl,
et de leurs sels pharmacologiquement acceptables,
caractérisé en ce qu'on dépose une préparation de glycosaminoglycanes d'héparine au sommet d'une colonne de chromatographie renfermant le facteur de croissance anionique ou cationique fixé à un support d'agarose, cette colonne étant équilibrée à l'aide d'un tampon de force ionique correspondant à une concentration de 0,2 M de NaCl, et en ce qu'on élue la fraction oligosaccharidique retenue sur la colonne en ajustant le tampon à une force ionique correspondant à une concentration de 1 M à 2 M de NaCl.

2. Procédé d'obtention d'oligosaccharides selon la revendication 1, caractérisé en ce qu'on soumet une préparation de glycosaminoglycanes d'héparine à une chromatographie d'échange d'ions sur une colonne échangeuse d'anions basiques forts tels que des ammoniums quaternaires, on fait varier la force ionique du tampon d'élution selon un gradient correspondant à une variation de force ionique d'un tampon de NaCl de 0,5 ± 0,1 M à 1-2 M, et on récupère les fractions les plus anioniques.

3. Procédé de préparation d'une composition pharmaceutique consistant à mettre un oligosaccharide tel que défini à la revendication 1 sous une forme pharmaceutiquement acceptable.

## Claims (Claims for the following Contracting State(s): BE, CH, DE, FR, GB, GR, IT, LI, LU, NL, SE)

1. Oligosaccharides, characterised in that they correspond to the following formula III:
R(GH)ₙR' III
in which
- n is a number between 2 and 6
- G-H corresponds to a disaccharide sequence of the structure (L-iduronic acid 2-0-sulphate)-(D-glucosamine NH-sulphate 6-0-sulphate),
- G being an L-iduronic acid 2-0-sulphate radical,
- H being a D-glucosamine NH-sulphate 6-0-sulphate radical,
- R represents either a hydrogen atom or a G'-H disaccharide unit in which the G' radical is an α,β-unsaturated uronic acid radical,
- R' represents either a hydrogen atom or a G-H' disaccharide unit in which the H' radical is a 2,5-anhydromannitol or a 2,5-anhydromannonic acid radical,
it being understood that the meanings of R and R' are such that the total number of monosaccharide units of the oligosaccharides, including the modified units, does not exceed 14,
- capable of being fixed to anionic FGF, fixed to a chromatographic column, in a 0.01 M tris HCl buffer medium, pH 7.4, containing 0.2 M Na Cl,
and their pharmocologically acceptable salts.

2. Oligosaccharide according to claim 1, characterised in that it is composed of chains containing 6 monosaccharide units.

3. Hexasaccharide according to claim 2, characterised in that it corresponds to:
- a product contained in a fraction corresponding to that eluted from a volume of between 32 and 36 l in a gel filtration system in which 60 g of precipitate obtained by alcoholic precipitation of a nitrous depolymerisation mixture of heparin is taken up in 500 ml of 0.5 M NaCl solution and is loaded on to a 100 cm x 25 cm column containing 45 litres of agarose-acrylamide, equilibrated with a 0.5 N NaCl buffer, pH 6.0, and in which elution is carried out at a flow rate of 1500 ml/hour,
- this product being separated by affinity chromatography on anionic FGF fixed to an agarose support, using a 2.5 cm x 6.5 cm column containing 30 ml of anionic FGF fixed to an agarose support and equilibrated with a 0.01 M tris HCl buffer, pH 7.4, containing 0.2 M NaCl, on to which 300 mg of the above fraction in a solution of 60 ml of the same buffer is loaded, by elution with the aid of this buffer adjusted to 1 M NaCl and by precipitation of the oligosaccharide from the fraction collected with the aid of ethanol.

4. Oligosaccharides according to claim 1, characterised in that they are composed of chains containing 8 monosaccharide units.

5. Octosaccharide according to claim 4, characterised in that its corresponds to:
- a product contained in a fraction corresponding to that eluted from a volume of between 28 and 32 l in a gel filtration system in which 60 g of precipitate obtained by alcoholic precipitation of a nitrous depolymerisation mixture of heparin is taken up in 500 ml of 0.5 M NaCl solution and is loaded on to a 100 cm x 25 cm column containing 45 litres of agarose-acrylamide, equilibrated with a 0.5 N NaCl buffer, pH 6.0, and in which elution is carried out at a flow rate of 1500 ml/hour,
- this product being separated by affinity chromatography on anionic FGF fixed to an agarose support, using a 2.5 cm x 6.5 cm column containing 30 ml of anionic FGF on an agarose support and equilibrated with a 0.01 M tris HCl buffer, pH 7.4, containing 0.2 M NaCl, on to which 300 mg of the above fraction in a solution of 60 ml of the same buffer is loaded, by elution with the aid of this buffer adjusted to 1 M NaCl and by precipitation of the oligosaccharide from the fraction collected with the aid of ethanol.

6. Oligosaccharides according to claim 1, characterised in that they are composed of chains containing 10 monosaccharide units.

7. Decasaccharide according to claim 6, characterised in that it corresponds to:
- a product contained in a fraction corresponding to that eluted from a volume of between 25 and 28 litres in a gel filtration system in which 60 g of precipitate obtained by alcoholic precipitation of a nitrous depolymerisation mixture of heparin is taken up in 500 ml of 0.5 M NaCl solution and is loaded on to a 100 cm x 25 cm column containing 45 litres of agarose-acrylamide, equilibrated with a 0.5 M NaCl buffer, pH 6.0, and in which elution is carried out at a flow rate of 1500 ml/hour,
- this product then being separated by affinity chromatography on anionic FGF fixed to an agarose support, using a 2.5 cm x 6.5 cm column containing 30 ml of anionic FGF on an agarose support and equilibrated with a 0.01 M tris HCl buffer, pH 7.4, containing 0.2 M NaCl, on to which 300 mg of the above fraction in a solution of 60 ml of the same buffer is loaded, by elution with the aid of this buffer adjusted to 1M NaCl and by precipitation of the oligosaccharide from the fraction collected with the aid of ethanol.

8. Oligosaccharides according to claim 1, characterised in that they are composed of chains containing 12 monosaccharide units.

9. Procedure for the preparation of oligosaccharides according to any one of claims 1, 2, 4, 6 and 8, characterised in that a preparation of glycosaminoglycans of heparin is loaded on to the top of a chromatographic column containing the anionic or cationic growth factor fixed to an agarose support, this column being equilibrated with the aid of a buffer having an ionic strength corresponding to a concentration of 0.2 M NaCl, and the oligosaccharide fraction retained on the column is eluted by adjusting the buffer to an ionic strength corresponding to a concentration of 1 M to 2 M NaCl.

10. Procedure for the preparation of oligosaccharides according to any one of claims 1, 2, 4, 6 and 8, characterised in that a preparation of glycosaminoglycans of heparin is subjected to ion exchange chromatography on a strongly basic anion exchange column, such as the quaternary ammonium type, the ionic strength of the elution buffer is varied according to a gradient corresponding to a variation in the ionic strength of an NaCl buffer from 0.5 ± 0.1 M to 1-2 M, and the most anionic fractions are recovered.

11. Pharmaceutical compositions, characterised in that they contain in their active principle at least one oligosaccharide according to any one of claims 1 to 8 in association with a pharmaceutical excipient.

12. Agent which modulates the growth of cells in culture, characterised in that it contains an oligosaccharide according to any one of claims 1 to 8.

13. Biological reagent, characterised in that it is prepared from an oligosaccharide according to any one of claims 1 to 8.

## Claims (Claims for the following Contracting State(s): AT, ES)

1. Procedure for the preparation of oligosaccharides corresponding to the following formula III:
R(GH)ₙR' III
in which
- n is a number between 2 and 6
- G-H corresponds to a disaccharide sequence of the structure (L-iduronic acid 2-0-sulphate)-(D-glucosamine NH-sulphate 6-0-sulphate),
- G being an L-iduronic acid 2-0-sulphate radical,
- H being a D-glucosamine NH-sulphate 6-0-sulphate radical,
- R represents either a hydrogen atom or a G'-H disaccharide unit in which the G' radical is an α,β-unsaturated uronic acid radical,
- R' represents either a hydrogen atom or a G-H' disaccharide unit in which the H' radical is a 2,5-anhydromannitol or a 2,5-anhydromannonic acid radical,
it being understood that the meanings of R and R' are such that the total number of monosaccharide units of the oligosaccharides, including the modified units, does not exceed 14,
- capable of being fixed to anionic FGF, fixed to a chromatographic column, in a 0.01 M tris HCl buffer medium, pH 7.4, containing 0.2 M Na Cl,
and their pharmocologically acceptable salts,
characterised in that a preparation of glycosaminoglycans of heparin is loaded on to the top of a chromatographic column containing the anionic or cationic growth factor fixed to an agarose support, this column being equilibrated with the aid of a buffer having an ionic strength corresponding to a concentration of 0.2 M NaCl, and the oligosaccharide fraction retained on the column is eluted by adjusting the buffer to an ionic strength corresponding to a concentration of 1 M to 2 M NaCl.

2. Procedure for the preparation of oligosaccharides according to claim 1, characterised in that a preparation of glycosaminoglycans of heparin is subjected to ion exchange chromatography on a strongly basic anion exchange column, such as the quaternary ammonium type, the ionic strength of the elution buffer is varied according to a gradient corresponding to a variation in the ionic strength of an NaCl buffer from 0.5 ± 0.1 M to 1-2 M, and the most anionic fractions are recovered.

3. Procedure for the preparation of a pharmaceutical composition consisting in bringing an oligosaccharide as defined in claim 1 into a pharmaceutically acceptable form.

## Patentansprüche (Patentansprüche für folgende(n) Vertragsstaat(en): BE, CH, DE, FR, GB, GR, IT, LI, LU, NL, SE)

1. Oligosaccharide, **dadurch gekennzeichnet**, daß sie der folgenden Formel III entsprechen:
R(GH)ₙR' III
in der
- n eine Zahl mit einem Wert von 2 bis 6,
- G-H eine Disaccharidkette der Struktur (L-Iduronsäure-2-O-sulfat)-(D-Glucosamin-NH-sulfat-6-O-sulfat),
- G eine L-Iduronsäure-2-O-sulfat-gruppe,
- H eine D-Glucosamin-NH-sulfat-6-O-sulfat-gruppe,
- R entweder ein Wasserstoffatom oder eine Disaccharideinheit G'-H, worin die Gruppe G' für den Rest einer α,β-ungesättigten Uronsäure steht,
- R' entweder ein Wasserstoffatom oder eine Disaccharideinheit G-H', worin die Gruppe H' eine 2,5-Anhydromannitol- oder 2,5-Anhydromannonsäure-gruppe bedeuten,
mit der Maßgabe, daß die Bedeutungen von R und R' derart sind, daß die Gesamtzahl der Monosaccharideinheiten der Oligosaccharide einschließlich der modifizierten Einheiten höchstens 14 beträgt,
- die dazu fähig sind, sich in einem 0,01 M Tris-HCl-Puffer pH 7,4, 0,2 M NaCl an anionischen FGF, der an eine Chromatographiesäule fixiert ist, zu binden,
und deren pharmakologisch annehmbare Salze.

2. Oligosaccharid nach Anspruch 1, **dadurch gekennzeichnet**, daß es aus Ketten gebildet ist, die 6 Monosaccharideinheiten umfassen.

3. Hexasaccharid nach Anspruch 2, **dadurch gekennzeichnet**, daß es:
- einem Produkt entspricht, das in einer Fraktion enthalten ist, die jener entspricht, die zwischen einem Volumen von 32 bis 36 ℓ von einem Gelfiltratonssystem eluiert wird, auf das man in Lösung in 500 ml 0,5 M NaCl 60 g eines Niederschlags, den man durch alkoholische Ausfällung aus einer durch Nitrit-Depolymerisation von Heparin gewonnenen Mischung erhalten hat, auf eine Säule mit den Abmessungen 100 cm x 25 cm, die 45 Liter Agarose-Acrylamid enthält, das mit einem 0,5 M NaCl-Puffer, pH 6,0 ins Gleichgewicht gebracht worden ist, aufträgt, und die Elution mit einem Durchsatz von 1500 ml/Stunde bewirkt,
- welches Produkt durch Affinitätschromatographie über anionischem FGF, der an einem Agaroseträger fixiert ist, abgetrennt wird unter Verwendung einer Säule mit den Abmessungen 2,5 cm x 6,5 cm, die mit einem 0,01 M Tris-HCl-Puffer, pH 7.4 0,2 M NaCl ins Gleichgewicht gebracht worden ist und 30 ml an den Agaroseträger fixierten anionischen FGF enthält, auf welche Säule man 300 mg der oben beschriebenen Fraktion, gelöst in 60 ml des gleichen Puffers, aufträgt und mit diesem auf 1 M NaCl eingestellten Puffer eluiert und das Oligosaccharid der aufgefangenen Fraktion mit Hilfe von Ethanol ausfällt.

4. Oligosaccharide nach Anspruch 1, **dadurch gekennzeichnet**, daß sie aus Ketten gebildet sind, die 8 Monosaccharideinheiten umfassen.

5. Octasaccharid nach Anspruch 4, **dadurch gekennzeichnet**, daß es einem Produkt entspricht:
- einem Produkt entspricht, das in einer Fraktion enthalten ist, die jener entspricht, die zwischen einem Volumen von 28 bis 32 ℓ von einem Gelfiltratonssystem eluiert wird, auf das man in Lösung in 500 ml 0,5 M NaCl 60 g eines Niederschlags, den man durch alkoholische Ausfällung aus einer durch Nitrit-Depolymerisation von Heparin gewonnenen Mischung erhalten hat, auf eine Säule mit den Abmessungen 100 cm x 25 cm, die 45 Liter Agarose-Acrylamid enthält, das mit einem 0,5 N NaCl-Puffer, pH 6,0 ins Gleichgewicht gebracht worden ist, aufträgt, und die Elution mit einem Durchsatz von 1500 ml/Stunde bewirkt,
- welches Produkt durch Affinitätschromatographie über anionischen FGF, der an einem Agaroseträger fixiert ist, abgetrennt wird unter Verwendung einer Säule mit den Abmessungen 2,5 cm x 6,5 cm, die mit einem 0,01 M Tris-HCl-Puffer, pH 7,4 0,2 M NaCl ins Gleichgewicht gebracht worden ist und 30 ml an den Agaroseträger fixierten anionischen FGF enthält, auf welche Säule man 300 mg der oben beschriebenen Fraktion, gelöst in 60 ml des gleichen Puffers, aufträgt und mit diesem auf 1 M NaCl eingestellten Puffer eluiert und das Oligosaccharid der aufgefangenen Fraktion mit Hilfe von Ethanol ausfällt.

6. Oligosaccharide nach Anspruch 1, **dadurch gekennzeichnet**, daß sie aus Ketten gebildet sind, die 10 Monosaccharideinheiten umfassen.

7. Decasaccharid nach Anspruch 6, **dadurch gekennzeichnet**, daß es einem Produkt entspricht:
- einem Produkt entspricht, das in einer Fraktion enthalten ist, die jener entspricht, die zwischen einem Volumen von 25 bis 28 ℓ von einem Gelfiltratonssystem eluiert wird, auf das man in Lösung in 500 ml 0,5 M NaCl 60 g eines Niederschlags, den man durch alkoholische Ausfällung aus einer durch Nitrit-Depolymerisation von Heparin gewonnenen Mischung erhalten hat, auf eine Säule mit den Abmessungen 100 cm x 25 cm, die 45 Liter Agarose-Acrylamid enthält, das mit einem 0,5 N NaCl-Puffer, pH 6,0 ins Gleichgewicht gebracht worden ist, aufträgt, und die Elution mit einem Durchsatz von 1500 ml/Stunde bewirkt,
- welches Produkt durch Affinitätschromatographie über anionischen FGF, der an einem Agaroseträger fixiert ist, abgetrennt wird unter Verwendung einer Säule mit den Abmessungen 2,5 cm x 6,5 cm, die mit einem 0,01 M Tris-HCl-Puffer, pH 7,4 0,2 M NaCl ins Gleichgewicht gebracht worden ist und 30 ml an den Agaroseträger fixierten anionischen FGF enthält, auf welche Säule man 300 mg der oben beschriebenen Fraktion, gelöst in 60 ml des gleichen Puffers, aufträgt und mit diesem auf 1 M NaCl eingestellten Puffer eluiert und das Oligosaccharid der aufgefangenen Fraktion mit Hilfe von Ethanol ausfällt.

8. Oligosaccharide nach Anspruch 1, **dadurch gekennzeichnet**, daß sie aus Ketten gebildet sind, die 12 Monosaccharideinheiten umfassen.

9. Verfahren zur Herstellung der Oligosaccharide nach einem der Ansprüche 1, 2, 4, 6 und 8, **dadurch gekennzeichnet**, daß man ein Heparin-Glykosaminoglykanpräparat auf den Kopf einer Chromatographiesäule aufträgt, die an einen Agaroseträger fixierten anionischen oder kationischen Wachstumsfaktor umfaßt, welche Säule mit einem Puffer ins Gleichgewicht gebracht worden ist, dessen Ionenstärke einer NaCl-Konzentration von 0,2 M entspricht, und von der man die auf der Kolonne festgehaltene Oligosaccharidfraktion eluiert, in dem man den Puffer auf eine Ionenstärke einstellt, die einer NaCl-Konzentration von 1 M bis 2 M entspricht.

10. Verfahren zur Herstellung der Oligosaccharide nach einem der Ansprüche 1, 2, 4, 6 und 8, **dadurch gekennzeichnet**, daß man ein Heparin-Glykosaminoglykanpräparat einer Ionenaustauschchromatographie unterwirft auf einer Austauschersäule für stark basische Anionen, wie quaternäre Ammoniumionen, und die Ionenstärke des Elutionspuffers gemäß einem Gradienten variiert, der einer Variation der Ionenstärke des NaCl-Puffers von 0,5 ± 0,1 M bis 1 - 2 M entspricht, und die am stärksten anionischen Fraktionen gewinnt.

11. Pharmazeutische Zubereitungen, **dadurch gekennzeichnet**, daß sie in ihrem Wirkstoff mindestens ein Oligosaccharid nach einem der Ansprüche 1 bis 8 in Kombination mit einem pharmazeutischen Trägermaterial umfassen.

12. Mittel zur Modulierung des Zellwachstums in Kulturen, **dadurch gekennzeichnet**, daß es ein Oligosaccharid nach einem der Ansprüche 1 bis 8 umfaßt.

13. Biologisches Reagens, **dadurch gekennzeichnet**, daß es ausgehend von einem Oligosaccharid nach einem der Ansprüche 1 bis 8 hergestellt worden ist.

## Patentansprüche (Patentansprüche für folgende(n) Vertragsstaat(en): AT, ES)

1. Verfahren zur Herstellung von Oligosacchariden der folgenden Formel III:
R(GH)ₙR' III
in der
- n eine Zahl mit einem Wert von 2 bis 6,
- G-H eine Disaccharidkette der Struktur (L-Iduronsäure-2-O-sulfat)-(D-Glucosamin-NH-sulfat-6-O-sulfat),
- G eine L-Iduronsäure-2-O-sulfat-gruppe,
- H eine D-Glucosamin-NH-sulfat-6-O-sulfat-gruppe,
- R entweder ein Wasserstoffatom oder eine Disaccharideinheit G'-H, worin die Gruppe G' für den Rest einer α,β-ungesättigten Uronsäure steht,
- R' entweder ein Wasserstoffatom oder eine Disaccharideinheit G-H', worin die Gruppe H' eine 2,5-Anhydromannitol- oder 2,5-Anhydromannonsäure-gruppe bedeuten,
mit der Maßgabe, daß die Bedeutungen von R und R' derart sind, daß die Gesamtzahl der Monosaccharideinheiten der Oligosaccharide einschließlich der modifizierten Einheiten höchstens 14 beträgt,
- die dazu fähig sind, sich in einem 0,01 M Tris-HCl-Puffer pH 7,4, 0,2 M NaCl an anionischen FGF, der an eine Chromatographiesäule fixiert ist, zu binden,
und von deren pharmakologisch annehmbaren Salzen,
**dadurch gekennzeichnet**, daß man ein Heparin-Glykosaminoglykanpräparat auf den Kopf einer Chromatographiesäule aufträgt, die an einen Agaroseträger fixierten anionischen oder kationischen Wachstumsfaktor umfaßt, welche Säule mit einem Puffer ins Gleichgewicht gebracht worden ist, dessen Ionenstärke einer NaCl-Konzentration von 0,2 M entspricht, und von der man die auf der Kolonne festgehaltene Oligosaccharidfraktion eluiert, indem man den Puffer auf eine Ionenstärke einstellt, die einer NaCl-Konzentration von 1 M bis 2 M entspricht.

2. Verfahren zur Herstellung von Oligosacchariden nach Anspruch 1, **dadurch gekennzeichnet,** daß man ein Heparin-Glykosaminoglykanpräparat einer Ionenaustauschchromatographie unterwirft auf einer Austauschersäule für stark basische Anionen, wie quaternäre Ammoniumionen, und die Ionenstärke des Elutionspuffers gemäß einem Gradienten variiert, der einer Variation der Ionenstärke des NaCl-Puffers von 0,5 ± 0,1 M bis 1 - 2 M entspricht, und die am stärksten anionischen Fraktionen gewinnt.

3. Verfahren zur Herstellung einer pharmazeutischen Zubereitung, das darin besteht, ein Oligosaccharid, wie es in Anspruch 1 definiert ist, in eine pharmazeutisch annehmbare Form zu bringen.
